# EUROPEAN PATENT APPLICATION

(11) **EP 2 690 171 A1**
(43) Date of publication of application: **29.01.2014**
(21) Application number: 11861431.2
(22) Date of filing: 13.05.2011
(51) Int. Cl.: C12N 7/01, A01H 1/00, A01H 5/00, A01N 63/00, C12N 5/10, C12N 15/83, C12N 15/40

(54) **RECOMBINANT ALSV FOR PREVENTING PATHOGENIC VIRAL INFECTION OF PLANT**

(30) Priority: 24.03.2011 JP 2011066704
(71) Applicant: Incorporated National University Iwate University, Morioka-shi, Iwate 020-8550 (JP)
(72) Inventor: YOSHIKAWA, Nobuyuki, Morioka-shi Iwate 020-8550 (JP)
(74) Representative: Rigby, Barbara Nicole
(86) International application number: PCT/JP2011/061099
(87) International publication number: WO 2012/127697

(57) **Abstract**

The invention provides a recombinant apple latent spherical virus (ALSV). The recombinant ALSV contains a partial fragment of a genomic RNA or cDNA from one or more plant pathogenic viruses, and manifests an interference effect against these plant pathogenic viruses. The invention thus provides a novel means of defending against infection of a plant pathogenic virus, without the risk of becoming virulence in the course of proliferation.

## Description

### Technical Field

The present invention relates to recombinant ALSV useful as a component or the like of a protective agent against pathogenic infection of plants such as agricultural crops.

### Background Art

### 1. Apple Latent Spherical Virus

Apple latent spherical virus (ALSV) is a spherical virus, 25 nm in diameter, consisting of two segmented single-stranded RNAs (RNA1, RNA2) and three coat proteins (Vp25, Vp20, and Vp24) (NPL 1). ALSV, originally isolated from apple, has a relatively wide host range, and produces chlorosis symptoms in Chenopodium quinoa (quinoa). The virus also latently infects the model plant Arabidopsis, and a wide variety of other plant species, including Nicotiana plants (tobacco, Nicotiana benthamiana, occidentalis, glutinosa), tomato, Cucurbitaceae plants (cucumber, melon, zucchini, luffa), Fabaceae plants (soybean, azuki bean, pea), and rose family fruit tree plants (apple, European pear, Japanese pear, peach). The present inventors have constructed ALSV infectious cDNA clones, and successfully expressed a foreign gene in grass plants and apples using an ALSV vector having a foreign gene introducing site added between RNA2 cell-to-cell movement protein (MP) and Vp25 (for example, NPL 2). It has also been found that inoculation of a GFP (green fluorescent protein)-expressing tobacco with an ALSV vector created by incorporating GFP induces silencing of the GFP expression in the tabacco, and that quenching of the GFP fluorescence occurs first in the inoculated leaves in a circular pattern before it eventually disappears from the entire upper leaves (for example, NPL 3). There is also a finding that infection with an ALSV containing a part of plant endogenous genes, specifically phytoene desaturase (PDS) gene and sulfer (SU) gene, induces virus induced gene silencing (VIGS) in plants such as tobacco, Arabidopsis, soybean, and cucumber, and that the ALSV systemically induces VIGS in the infected plants (for example, NPL 4).

### 2. RNA Silencing

RNA silencing, first reported in 1990 in plants, is a sequence-specific gene deactivation mechanism universally conserved in eukaryotes (NPL 5), and is induced primarily by double-stranded RNA (dsRNA). Infection with an RNA virus causes the synthesis of dsRNA by the action of RNA-dependent RNA polymerase (RdRp), and the dsRNA is broken down into small interfering RNAs (siRNAs) of 21 to 25 nucleotides (nt) by the double-stranded RNA degrading enzyme Dicer (Dicer-like or DCL in plants) (NPL 6 and NPL 7). The siRNA is incorporated into the RNA-induced silencing complex (RISC), where an RNA having a nucleotide sequence complementary to the siRNA is degraded. The silencing systemically spreads as the siRNA moves to the adjacent cells and an RNA complementary to the siRNA is converted into dsRNA by the action of RdRp. RNA silencing has an important role as a defense mechanism against viruses in plants not endowed with an immune mechanism (NPL 8). It was reported in 1995 that infection with a TMV integrated with a plant endogenous gene induces RNA silencing of the gene (NPL 9). To date, the VIGS has been widely used as a reverse genetics technique.

### 3. Plant Pathogenic Virus, and Virus Control

Infection of cucurbits crops with zucchini yellow mosaic virus (ZYMV) has raised concerns worldwide over the damages it causes to the plants. Damages to cucumbers are also a growing problem in Japan. ZYMV is a filamentous virus of the genus Potyvirus, consisting of a single-stranded RNA of about 9,600 nt and measuring about 750 nm in length. ZYMV is aphid borne, and spreads by non-persistent transmission. The virus is known to cause symptoms such as severe mosaic, deformation in leaves and fruits, and atrophy in plants, particularly in cucurbits (such as cucumbers, squash, melons, and watermelons) (NPL 10 and NPL 11).

ZYMV, squash mosaic virus of the genus Potyvirus (watermelon mosaic virus 2: WMV2), and cucumber mosaic virus of the genus Cucumovirus (CMV) are regarded as major pathogens of cucumber mosaic disease, and cause the disease by single infection or superinfection (NPL 11). CMV is a type member of the genus Cucumovirus, spherical in shape with a diameter of 29 nm, and consisting of segmented three single-stranded RNAs (RNA1, RNA2, and RNA3) and a subgenomic RNA (RNA4). The host and pathogenicity of CMV vary among different varieties, and some members of CMV have satellite RNA. CMV is non-persistently transmitted by aphids, and has a very wide host range, infecting more than 1,000 plant species (NPL 10).

In order to prevent damages to agricultural crops caused by ZYMV and CMV, there have been attempts to take advantage of the interference effect of weakly pathogenic ZYMV and CMV (attenuated ZYMV, attenuated CMV), in addition to using resistant varieties, and controlling aphids (NPL 12 and NPL 13). The interference effect is an old phenomenon first discovered in tobacco mosaic virus in 1929, describing a phenomenon in which a plant infected with one virus is protected against further infection with the same virus. The two most likely mechanisms of the interference effect are virus coat proteins (CP)-mediated interference, and RNA-mediated interference (NPL 14 and NPL 15).

PTL 1 and PTL 2 describe inventions concerning attenuated virus as a means of defending against ZYMV and CMV. PTL 3 discloses a combined attenuated virus obtained by combining two or more attenuated viruses of the same virus group. PTL 4 and PTL 5 describe techniques to make an attenuated virus by incorporating a virus satellite RNA into the associated virus. In Japan, an attenuated ZYMV prepared as a biological agrichemical is commercially available, and has been used for cucumbers (NPL 7 and NPL 12). Tomato seedlings infected with an attenuated CMV having a satellite RNA are also available in the market (NPL 16).

However, no technique is known in which a recombinant virus created by incorporating a pathogenic virus gene into a different non-pathogenic vector virus is used as a means of defending against pathogenic viral infection.

### Citation List

### Patent Literature

PTL 1: JP-A-5-68540
PTL 2: JP-A-10-203901
PTL 3: JP-A-2000-264806
PTL 4: JP-A-5-3789
PTL 5: JP-A-11-279013

### Non Patent Literature

NPL 1: Li, C., Yoshikawa, N., Takahashi, T., Ito, T., Yoshida, K. and Koganezawa, H. (2000). Nucleotide sequence and genome organization of apple latent spherical virus: a new virus classified into the family Comoviridae. J. Gen. Virol. 81:541-547.
NPL 2: Li, C., Sasaki, N., Isogai, M. and Yoshikawa, N. (2004). Stable expression of foreign proteins in herbaceous and apple plants using Apple latent spherical virus RNA2 vectors. Arch. Virol. 149:1541-1558.
NPL 3: Yaegashi, H., Yamatsuta, T., Takahashi, T., Li, C., Isogai, M., Kobori, T., Ohki, S. and Yoshikawa, N. (2007). Characterization of virus-induced gene silencing in tobacco plants infected with apple latent spherical virus. Arch. Virol. 152:1839-1849.
NPL 4: Igarasi, A., Yamagata, K., Sugai, T., Takahashi, Y., Sugawara, E., Tamura, A., Yaegashi, H., Yamagishi, N., Takahashi, T., Isogai, M., Takahashi, H. and Yoshikawa, N. (2009). Apple latent spherical virus vectors for reliable and effective virus-induced gene silencing among abroad range of plants including tobacco, tomato, Arabidopsis thaliana, cucurbits, and legumes. Virology 386:407-416.
NPL 5: Hamiltom, A., Voinnet, O., Chappell, L. and Baulcombe, D. (2002). Two classes of short interfering RNA in RNA silencing. EMBO. J. 21:4671-4679.
NPL 6: Kosaka, Y. and Fukunishi, T. (1997). Multiple Inoculation with Three Attenuated Viruses for the Control of Cucumber Virus Disease. Plant Dis. 81:733-738.
NPL 7: Voinnet O. (2001). RNA silencing as a plant immune system against viruses. Trends Genet. 17:449-459.
NPL 8: Kumagai, M.H., Donson, J., Della-Cioppa, G., Harvey, D., Hanley, K. and Grill, L.K. (1995). Cytoplasmic inhibition of carotenoid biosynthesis with virus-derived RNA. Proc. Natl. Acad. Sci. USA 92:1679-1683.
NPL 9: Shoichi Hatanaka, ed. (1997), Virus Gaku, Asakura Publishing Co., Ltd., pp.427-434, 461-466.
NPL 10: Kunihei, Kishi, ed. (1988), Sakumotsu Byogai Jiten, Zenkoku Noson Kyoiku Kyokai, pp.321-322
NPL 11: Kosaka, Y., Ryang, Bo-Song., Kobori, T., Shiomi, H., Yasuhara, H. and Kataoka, M. (2006). Effectiveness of an Attenuated Zucchini yellow mosaic virus Isolate for Cross-Protecting Cucumber. Plant Dis. 90:67-72.
NPL 12: Wang, Wei-Qin., Natsuaki, T. and Kosaka, Y. (2006). Comparison of the nucleotide and amino acid sequences of parental and attenuated isolates of Zucchini yellow mosaic virus. J. Gen. Plant Pathol. 72:52-56.
NPL 13: Beachy, R.N. (1999). Coat-protein-mediated resistance to tobacco mosaic virus: discovery mechanisms and exploitation. Philos. Trans. R. Soc. Lond. B. Biol. Sci. 354:659-664.
NPL 14: Lin, S-S., Henriques, R., Wu, H-W., Niu, Q-W., Yen, S-D. and Chua, N-H. (2007). Strategies and mechanisms of plant virus resistance. Plant Biotechnol. Rep. 1:125-134.
NPL 15: Kazuhisa Tsuda, Yoshitaka Kosaka, Takashi Kobori, Hiroshi Shiomi, Keiichi Musumi, Mitsunobu Kataoka (2005). Effects of Fertilizer Application on Yield and Vitamin C Content of Tomato Inoculated with the Attenuated Isolate CM95 of Cucumber Mosaic virus, Japanese Journal of Phytopathology 71:1-5.

### Summary

### Technical Problem

As described above, plant protection against pathogenic viruses using the interference effect of attenuated virus is known. However, the method using an attenuated virus is limited to only some viruses for which attenuated viruses are available, and is not easily applicable when isolation of an attenuated virus from nature is difficult, or when an attenuated virus cannot be easily created by mutation. Further, the interference effect by an attenuated virus does not manifest itself against different viruses, and each virus requires a different attenuated virus. Creating an attenuated virus, however, is time consuming and very laborious.

In the case of attenuated viruses that involve insertion of satellite RNA, the parental virus is pathogenic, and there is always a risk of reversion to virulence in the course of proliferation within a plant.

The present invention was made under these circumstances, and an object of the present invention is to provide a novel means of creating a virus that manifests the interference effect against theoretically any pathogenic viruses, without the risk of becoming virulence in the course of proliferation.

### Solution to Problem

As a solution to the foregoing problems, there is provided a recombinant apple latent spherical virus (ALSV) comprising a partial fragment of a genomic RNA or cDNA from one or more plant pathogenic viruses.

It is preferable in the recombinant ALSV that the plant pathogenic virus be a zucchini yellow mosaic virus (ZYMV), a cucumber mosaic virus (CMV), or a soybean mosaic virus (SMV).

This application also provides a method for defending against infection of a plant pathogenic virus, which comprises inoculating the recombinant ALSV into a seedling of a plant, and a pathogenic virus-resistant plant, which is inoculated with the recombinant ALSV in a seedling stage.

### Advantageous Effects of Invention

The present invention defends against infection of a plant pathogenic virus with the use of a recombinant ALSV that contains a partial fragment of a genomic RNA or cDNA from the plant pathogenic virus of interest. The pathogenic virus is not limited, and it is theoretically possible to create a recombinant ALSV that can defend against infection of any pathogenic virus. A person ordinary skilled in the art would be able to easily create the recombinant ALSV by referring to the descriptions contained in this application, with much less time and effort than those required for conventional attenuated viruses.

ALSV is a latently infectious virus, and a partial fragment of the genomic RNA or cDNA of a pathogenic virus inserted into the ALSV is not pathogenic itself. Accordingly, there is no risk of the recombinant ALSV acquiring virulence within a plant, and the recombinant ALSV is safe to use.

### Brief Description of Drawings

FIG. 1 represents configurations of ALSV infectious cDNA clones and an ALSV vector. pEALSR1: ALSV RNA1 infectious cDNA clone, pEALSR2L5R5: ALSV RNA2 infectious cDNA clone, P35S: 35S promoter, Tnos: nopaline synthase terminator, Q/G: protein cutting site, PRO-co: protease cofactor, HEL: NTP-binding helicase, C-PRO: cystein protease, POL: RNA polymerase, MP: movement protein, VP25, VP20, VP24: capsid protein.
FIG. 2 represents the steps of constructing ALSV vectors through introduction of ZYMV gene fragments in Example 1.
FIG. 3 shows the locations of the leaf discs collected in Example 1.
FIG. 4 represents the results of Example 1, showing ZYMV disease symptoms (21 dpi) in the first true leaf secondary inoculation group of a cucumber (variety: Aodai). Upper left: (A) fifth true leaf; bottom left: (B) sixth true leaf; right: (C) whole plant. 1: non-inoculated group, 2: ALSV only group, 3: ZYMV only group, 4: wtALSV+ZYMV group, 5: ALSV-Z:CP200+ZYMV group (from the top left to bottom right in (A) and (B); from the left in (C)).
FIG. 5 represents the results of Example 1, showing the GFP fluorescence distributions (14 dpi) of the first to sixth true leaves in the ZYMV only group.
FIG. 6 represents the results of Example 1, showing the GFP fluorescence distributions (14 dpi) of the first to sixth true leaves in the wtALSV+ZYMV group.
FIG. 7 represents the results of Example 1, showing the GFP fluorescence distributions (14 dpi) of the first to sixth true leaves in the ALSV-Z:CP200+ZYMV group.
FIG. 8 represents the results of Example 1, analyzing the ZYMV and ALSV accumulation levels by direct ELISA in the first true leaf secondary inoculation group of a cucumber (variety: Aodai).
FIG. 9 represents the results of Example 1, analyzing the ZYMV and ALSV accumulation levels by direct ELISA in the second true leaf secondary inoculation group of a cucumber (variety: Aodai).
FIG. 10 represents the results of Example 1, analyzing the ZYMV and ALSV accumulation levels by direct ELISA in the third true leaf secondary inoculation group of a cucumber (variety: Aodai).
FIG. 11 represents the results of Example 1, showing disease symptoms (21 dpi) in each test group of a cucumber (variety: Aodai) that had primary inoculation of ALSV vectors containing different ZYMV gene fragments. Upper left: (A) fifth true leaf; bottom left: (B) sixth true leaf; right: (C) whole plant. 1: non-inoculated group, 2: ALSV only group, 3: ZYMV only group, 4: wtALSV+ZYMV group, 5: ALSV-Z:P1+ZYMV group, 6: ALSV-Z:P3+ZYMV group, 7: ALSV-Z:CP200+ZYMV group (from the top left to bottom right in (A) and (B); from the left in (C)).
FIG. 12 represents the results of Example 1, showing GFP fluorescence distributions (14 dpi) of the first to sixth true leaves in the ALSV-Z:P3+ZYMV group.
FIG. 13 represents the results of Example 1, showing GFP fluorescence distributions (14 dpi) of the first to sixth true leaves in the ALSV-Z:P1+ZYMV group.
FIG. 14 represents the results of Example 1, analyzing the ZYMV and ALSV accumulation levels by direct ELISA in the first true leaf secondary inoculation group of a cucumber (variety: Aodai) that had primary inoculation of ALSV vectors containing different ZYMV gene fragments.
FIG. 15 represents the results of Example 1, analyzing the ZYMV and ALSV accumulation levels by direct ELISA in the first true leaf secondary inoculation group of a cucumber (variety: Suzunari Suyo).
FIG. 16 represents the results of Example 1, analyzing the ZYMV and ALSV accumulation levels by direct ELISA in the first true leaf secondary inoculation group of a cucumber (variety: Tsubasa).
FIG. 17 represents the results of Example 1, showing disease symptoms (21 dpi) in each test group of a cucumber (variety: Aodai) that had primary inoculation of ALSV vectors containing ZYMV gene fragments of different lengths. Upper left: (A) fifth true leaf; bottom left: (B) sixth true leaf; right: (C) whole plant. 1: non-inoculated group, 2: ALSV only group, 3: ZYMV only group, 4: wtALSV+ZYMV group, 5: ALSV-Z:CP100+ZYMV group, 6: ALSV-Z:CP200+ZYMV group (from the top left to bottom right in (A) and (B); from the left in (C)).
FIG. 18 represents the results of Example 1, showing GFP fluorescence distributions (14 dpi) of the first to sixth true leaves in the ALSV-Z:CP100+ZYMV group.
FIG. 19 represents the results of Example 1, analyzing the ZYMV and ALSV accumulation levels by direct ELISA in the first true leaf secondary inoculation group of a cucumber (variety: Aodai) that had primary inoculation of ALSV vectors containing ZYMV gene fragments of different lengths.
FIG. 20 represents the results of Example 1, showing disease symptoms (21 dpi) in each test group of a cucumber (variety: Aodai) that had primary inoculation of attenuated ZYMV and various ALSVs containing different ZYMV gene fragments. (A), fifth true leaf; (B), whole plant. 1: non-inoculated group, 2: ALSV only group, 3: ZYMV only group, 4: wtALSV+ZYMV group, 5: ALSV-Z:P1+ZYMV group, 6: ALSV-Z:P3+ZYMV group, 7: ALSV-Z:CP200+ZYMV group, 8: ZYMV 2002+ZYMV group (from the left to right in (A) and (B)).
FIG. 21 represents the results of Example 1, showing GFP fluorescence distributions (14 dpi) of the first to sixth true leaves in the ZYMV 2002+ZYMV group.
FIG. 22 represents the results of Example 1, analyzing the ZYMV and ALSV accumulation levels by direct ELISA in the first true leaf secondary inoculation group of a cucumber (variety: Aodai) that had primary inoculation of attenuated ZYMV and various ALSVs containing different ZYMV gene fragments.
FIG. 23 represents the CMV-Y gene cloning steps, and the steps of constructing CMV-Y gene fragments in Example 2.
FIG. 24 represents the steps of constructing ALSV vectors containing CMV-Y gene fragments in Example 2.
FIG. 25 represents the results of Example 2, showing CMV-Y disease symptoms (9 dpi) of a tobacco that had primary inoculation of ALSV vectors containing CMV-Y gene fragments. From the upper left to the bottom right of the figure, non-inoculated group, CMV-Y only group, ALSV only group, wtALSV+CMV-Y group, ALSV-C:1a+CMV-Y group, ALSV-C:2a+CMV-Y group, ALSV-C:CP+CMV-Y group.
FIG. 26 represents the results of Example 2, analyzing the CMV-Y and ALSV accumulation levels by direct ELISA in a tobacco that had primary inoculation of various ALSVs containing different CMV-Y gene fragments.
FIG. 27 represents the results of Example 2, showing CMV-Y disease symptoms (18 dpi) of a cucumber (variety: Aodai) that had primary inoculation of ALSV vectors containing CMV-Y gene fragments. (A), whole plant; (B), sixth true leaf. 1: non-inoculated group, 2: ALSV only group, 3: CMV-42CM only group, 4: wtALSV+CMV-42CM group, 5: ALSV-C:1a+CMV-42CM group, 6: ALSV-C:2a+CMV-42CM group, 7: ALSV-C:CP+CMV-42CM group (from the left in (A); from the upper left to the bottom right in (B)).
FIG. 28 represents the results of Example 2, analyzing the CMV-42CM and ALSV accumulation levels by direct ELISA in the first true leaf secondary inoculation group of a cucumber (variety: Aodai) that had primary inoculation of ALSV vectors containing CMV-Y gene fragments.

### Description of Embodiments

The recombinant ALSV of the present invention includes an inserted partial fragment of the genomic RNA or cDNA of a plant pathogenic virus. Upon replication of the recombinant ALSV, siRNA is synthesized that contains the sequence of the inserted fragment, and a region of a pathogenic virus genome complementary to the siRNA is disrupted in accordance with the principle of RNA silencing.

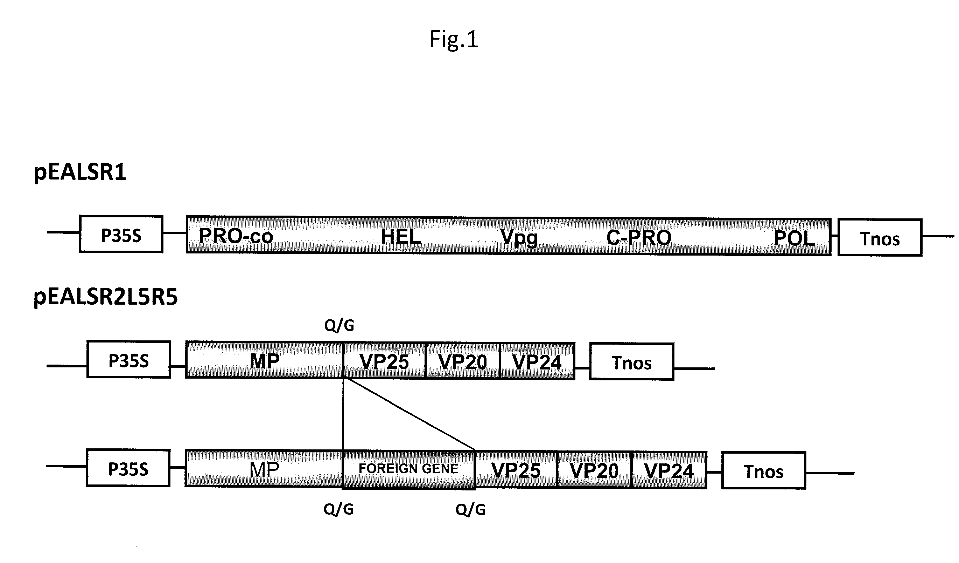

The ALSV may be isolated from apple, or, more preferably, ALSV vectors such as the ALSV RNA1 infectious cDNA clone (pEALSR1), and the RNA2 infectious cDNA clone (pEALSR2L5R5) described in PTL 2 may be used (see FIG. 1).

The plant pathogenic virus may be, in principle, any virus, including the cucumber mosaic virus (CMV), zucchini yellow mosaic virus (ZYMV), and soybean mosaic virus (SMV) used in Examples. Examples include squash mosaic virus (WMV-2), broad bean wilt virus (BBWV), and turnip mosaic virus (TuMV) transmitted by aphids as with the case of CMV and ZYMV; tomato spotted wilt virus (TSWV), impatiens necrotic spot virus (INSV), and iris yellow spot virus (IYSV) transmitted by thrips; cucumber yellows virus (CuYV) transmitted by whiteflies; and tomato mosaic virus (ToMV) transmitted through soil.

The recombinant ALSV of the present invention may be one in which partial fragments of the genomic RNA or cDNA of one or more (for example, three) pathogenic viruses are inserted into an ALSV vector in combination (for example, in the form of a fused polynucleotide). Such a recombinant ALSV against multiple pathogenic viruses has a combination vaccine-like function, and can protect the same plant individual from infection by multiple pathogenic viruses. For example, a cucumber can be protected from infection by zucchini yellow mosaic virus (ZYMV) and cucumber mosaic virus (CMV), as will be described in Examples.

The inserted fragment in the ALSV is prepared from the genomic RNA or cDNA of a pathogenic virus. The inserted fragment may be selected from any region of a pathogenic virus genome, preferably from gene regions associated with survival, proliferation, or pathogenicity of the pathogenic virus. For example, as will be described in Examples, partial regions of genomic RNA or corresponding cDNA are selected that encode P1, HC-Pro, P3, CI, NIa, NIb, CP (preferably, P3, CI, Nla, NIb, CP) in the case of zucchini yellow mosaic virus (ZYMV), 1 a, 2a, CP (preferably 2a) in the case of cucumber mosaic virus (CMV), and CP in the case of soybean mosaic virus (SMV).

The inserted fragment may have any size, as long as the inserted fragment, for example, in an ALSV vector does not affect the infection or proliferation in the plant individual. The inserted fragment is sized so that a siRNA containing the inserted fragment sequence effectively disrupts the pathogenic virus genome, and that the inserted fragment is stably maintained. Specifically, the inserted fragment size may be, for example, about 100 to 300 bp, preferably about 150 to 200 bp.

It should be noted that frame matching is necessary so as to allow the coded peptide of the inserted fragment to be expressed as a fused peptide with the ALSV peptide (i.e., a stop codon does not occur after the insertion of the partial fragment). When using an ALSV vector, a partial fragment is prepared with restriction enzymes that match the cloning sites, and inserted into the ALSV vector with a commercially available ligation kit or the like. The recombinant ALSV vector is then used to infect a quinoa, and viruses are isolated from the infected leaves to obtain the recombinant ALSV.

The recombinant ALSV may directly be inoculated to a plant to prevent pathogenic virus infections. Alternatively, the recombinant ALSV may be prepared into an infection preventing agent by being freeze dried in a buffer containing a stabilizer or the like (for example, PTL 2).

The recombinant ALSV, or the dissolved infection preventing agent may be inoculated to the leaves in a seedling of a plant (for example, to the developing cotyledon of a cucumber, and to the third to fifth true leaves in 5 leaf stage of a tobacco) by using known methods such as the silicon carbide method.

The present invention is described below more specifically in greater detail using Examples. It should be noted, however, that the present invention is not limited by the following descriptions.

### Example 1

Interference Effect by ALSV Vector Containing ZYMV Gene Fragment

### 1: Materials and Methods

### 1-1: Experiment Materials

### 1-1-1: Test Plant

A Chenopodium quinoa (hereinafter, "quinoa") plant was used for proliferating ALSV. For interference effect assay, three varieties (Aodai cucumber, Suzunari Suyo, and Tsubasa) of Cucumis sativus (hereinafter, "cucumber") with no ZYMV resistance were used.

### 1-1-2. Test Infectious Clone

The ALSV RNA1 infectious cDNA clone (pEALSR1) and RNA2 infectious cDNA clone (pEALSR2L5R5)(FIG. 1) described in NPL 2 and elsewhere, and the infectious cDNA clone (p35S-Z5 vector+GFP-FL) of lethal strain ZYMV isolate Z5-1 (accession number: AB188115; a kind gift from Dr. Tomohide Natsuaki at Utsunomiya University, Faculty of Agriculture) were used as test infectious clones. The attenuated ZYMV isolate 2002 (hereinafter, "ZYMV 2002"; accession number: AB188116; a kind gift from Dr. Ryo Hosei at the Institute of Microbial Chemistry) was also used.

### 1-2: Introduction of ZYMV Gene Fragment into ALSV Vector

### 1-2-1: Cloning of ZYMV Gene Fragment

Primers were designed from the known P1, P3, and CP gene sequences of ZYMV isolate Z5-1, using p35S-Z5 vector+GFP-FL as a template. Specifically, the following primer sets were used for amplification (Table 1; SEQ ID NOS: 1 to 7).
Z:P1-Xho(+) and Z:P1-Bam(-) for P1 gene (210 nt)
Z:P3-Xho(+) and Z:P3-Bam(-) for P3 gene (189 nt)
Z:CP-Xho(+) and Z:CP-Bam(-) for CP gene (225 nt)
Z:CP-Xho(+) and Z:CP100-Bam(-) for CP gene (117 nt)

**Table 1**

| Primer sequences used for introduction of ZYMV gene fragment into ALSV vector | |
|---|---|
| Primer | Sequence (5'-> 3') |
| Z:P1-Xho(+) | TACATCTCGAG₂₀₉GCAAGTAGTCGGGTTGGTATA₂₃₀ |
| Z:P1-Bam(-) | TACATGGATCC₄₁₈TCCATTCGGACCCTTTTTCACC₃₉₇ |
| Z:P3-Xho(+) | TACATCTCGAG₃₂₆₉GCGGCGACAAGGGAAGAAAAA₃₂₈₉ |
| Z:P3-Bam(-) | TACATGGATCC₃₄₅₇TGCGCCAGCCATGTACAAG₃₄₃₉ |
| Z:CP-Xho(+) | TACATCTCGAG₈₆₂₇GGCTCCGGCTCAGGTGAGAAAA₈₆₄₈ |
| Z:CP-Bam(-) | TACATGGATCC₈₈₅₁AGAGGCGAATTGCTGATGAG₈₈₃₂ |
| Z:CP100-Bam(-) | TACATGGATCC₈₇₄₃CAATGACATTTTCTTTGTGA₈₇₂₄ |

Restriction enzyme recognition sequences are underlined.

A 2-µl template DNA solution (1 ng/µl) was placed in a 0.2 ml-volume PCR tube, and mixed with sterile water (35 µl), 10 x Ex Taq Buffer (TaKaRa; 5 µl), 2.5 mM dNTP Mixture (TaKaRa; 5 µl), 10 µM gene fragment amplification primers (1 µl each), and TaKaRa Ex Taq™ (TaKaRa; 1 µl) added to the tube. A PCR was performed at 94°C for 5 min using a TaKaRa PCR Thermal Cycler Dice Version III Model TP600 (TaKaRa), followed by 30 cycles of reaction [94°C, 30 seconds → 57°C, 30 seconds → 72°C, 60 seconds], and at 72°C for 5 minutes, and 4°C for 5 minutes. The resulting PCR product was electrophoresed, as follows. A 1% agarose gel prepared from Agarose S (Nippon Gene; 0.15 g), 15 ml of TAE [40 mM Tris, 40 mM acetic acid, 1 mM EDTA (pH 8.0)], and ethidium bromide (0.6 µl) was installed in a Mupid-2 plus (ADVANCE) filled with TAE in the electrophoresis chamber. The PCR product (2 µl) was mixed with 1 µl of a 10 x Loading Buffer (TaKaRa), and applied to agarose gel wells, and electrophoresed to confirm that the product was a fragment of the designed size.

The PCR product of the intended size was purified by using a MonoFas DNA purification kit I (GL Sciences), as follows. A band of the PCR product (20 µl) electrophoresed on 1 % agarose was cut with a surgical knife, and placed in a 1.5 ml-volume microtube. After weighing the gel, an equal amount of Buffer A was added, and the mixture was kept at the maintained temperature of 60°C for 10 minutes to completely dissolve the gel. The dissolved gel sample solution was charged into a spin column fitted with a collection tube, and centrifuged at 10,000 rpm (4°C) for 30 seconds. After the centrifugation, 500 µl of Buffer B was added to the spin column, and the mixture was centrifuged at 10,000 rpm (4°C) for 30 seconds. After the centrifugation, the spin column was attached to a 1.5 ml-volume microtube, and centrifuged at 10,000 rpm (4°C) for 60 seconds after adding 20 µl of Buffer C. The resulting solution was obtained as each ZYMV gene fragment.

Each gene fragment was TA cloned by using a pGEM-T Easy Vector System (Promega), as follows. The P1 gene fragment (4 µl) was placed in a 1.5 ml-volume microtube, and a 2 x Rapid Ligation Buffer (5 µl), pGEM-T Easy Vector (1 µl), and T4 DNA Ligase (1 µl) were added. These were mixed, and allowed to stand at room temperature for 60 minutes for ligation reaction. After the ligation reaction, the sample solution (10 µl) was added to the competent cells of Escherichia coli DH5α (100 µl) thawed beforehand on ice. After being gently mixed, the mixture was allowed to stand on ice for 20 minutes. This was followed by 45 seconds of heat shock in a 42°C water bath, and the sample was immediately allowed to stand on ice for 2 minutes. Thereafter, 1 ml of a SOC medium (pH7.5) [containing bacto tryptone (20 g), bacto yeast extract (5 g), NaCl (0.5 g), 1 M MgCl₂ (10 ml), and 1 M MgSO₄ (10 ml) per liter] was added to the sample, and the mixture was shake cultured for 60 minutes in a 37°C shaker. After culture, the sample (50 µl) was spread over a 0.15% agar LB medium (pH 7.5) plate [containing bacto tryptone (10 g), bacto yeast extract (5 g), NaCl (10 g), and 2 ml of ampicillin (25 mg/ml) per liter]. The sample plate was allowed to stand for 14 to 16 hours in a 37°C incubator.

The established colonies on the plate were grown in small-scale culture, and plasmids were extracted by using a method that involved boiling, as follows. The cells were inoculated with an autoclaved toothpick to LB medium (2 ml) dispensed in a test tube, and shake cultured for 8 hours in a 37°C shaker. The culture medium was transferred to a 1.5 ml-volume microtube, and centrifuged at 14,000 rpm (room temperature) for 1 minute. After the centrifugation, the supernatant was removed with an aspirator, and 350 µl of STET [0.1 M NaCl, 10 mM Tris-HCl (pH 8.0), 1 mM EDTA (pH8.0), and 5% Triton X-100] was added to suspend the precipitate with a vortex mixer. Then, 25 µl of a lysozyme solution [10 mM Tris-HCl (pH 8.0), 10 mg/ml lysozyme] was added, and mixed for 3 seconds with a vortex mixer. The microtube was then boiled for 40 seconds, and immediately placed in ice to cool for 5 minutes. After being cooled, the microtube was centrifuged at 14,000 rpm (room temperature) for 10 minutes, and the precipitate was removed with an autoclaved toothpick. Then, 40 µl of 3 M sodium acetate (pH 5.2) and 420 µl of isopropyl alcohol were added and mixed with the supernatant, and the mixture was allowed to stand for 5 minutes in a -20°C freezer. The mixture was then centrifuged at 14,000 rpm (4°C) to remove the supernatant. After adding 1 ml of 70% ethanol to the precipitate in the microtube, the mixture was centrifuged at 14,000 rpm (4°C) for 2 minutes, and the supernatant was removed. The precipitate in the microtube was dried for 2 minutes with a vacuum drier, and the dried precipitate was suspended in 50 µl of an RNase solution [10 mM Tris-HCl (pH 8.0), 1 mM EDTA (pH 8.0), and 20 µg/ml DNase free RNase]. The suspension was allowed to stand for 20 minutes in a 37°C incubator to obtain a plasmid solution.

The extracted plasmid solution (2 µl) was electrophoresed on 1 % agarose gel to screen for plasmids with possible inserts. The screened plasmids were treated with restriction enzyme EcoRI to check for the retention of the inserts, as follows. The plasmids (2 µl) were placed in a microtube, and sterile water (6.8 µl), 10 x H Buffer (TaKaRa; 1 µl), and EcoRI (TaKaRa; 0.2 µl) were added and mixed. The mixture was allowed to stand for 3 hours in a 37°C incubator. This was followed by 1 % agarose gel electrophoresis to check for the retention of the inserts. This completed the TA cloning.

### 1-2-2: ZYMV Gene Fragment Sequence

The plasmids obtained by TA cloning were purified according to the following method. TE [10 mM Tris-HCl (pH 8.0), 1 mM EDTA (pH 8.0)] (154 µl), TE saturated phenol (100 µl), and chloroform (100 µl) were added and mixed with the plasmids (46 µl) for 5 minutes with a vortex mixer, and the mixture was centrifuged at 14,000 rpm (4°C) for 5 minutes. After the centrifugation, the aqueous layer (200 µl) was transferred to a new microtube, mixed with 200 µl of chloroform with a vortex mixer, and centrifuged at 14,000 rpm (4°C) for 5 minutes. The aqueous layer (150 µl) was transferred to a new microtube, and mixed with 3 M sodium acetate (pH 5.2; 15 µl) and 99% ethanol (375 µl) for 2 minutes with a vortex mixer. The mixture was allowed to stand for 20 minutes in a -20°C freezer, and centrifuged at 14,000 rpm (4°C) for 20 minutes to remove the supernatant. After adding 70% ethanol (1 ml) to the precipitate, the mixture was centrifuged at 14,000 rpm (4°C) for 5 minutes. The supernatant was removed after the centrifugation, and the precipitate was dried for 2 minutes with a vacuum drier, and suspended in 50 µl of TE. After adding and mixing 50 µl of PEG solution (13% polyethylene glycol 8000, 1.6 M NaCl), the mixture was allowed to stand for 1 hour on ice. The mixture was centrifuged at 14,000 rpm (4°C) for 20 minutes. After adding 70% ethanol (500 µl) to the precipitate, the mixture was centrifuged at 14,000 rpm (4°C) for 2 minutes. After removing the supernatant, the precipitate was dried for 2 minutes with a vacuum drier. The precipitate was suspended in 20 µl of sterile water, and the concentration was adjusted to 300 ng/µl by measuring absorbance (wavelength 260 nm) with a NanoDrop ND-1000 Spectrophotometer (Asahi Techno Glass). The resulting product was obtained as the purified plasmid.

The sequence of the purified plasmid was analyzed according to the method described below. The electrophoresis sample used for the sequence analysis was prepared by using a BigDye Terminator v3.1 Cycle Sequencing Kit (Applied Biosystems), as follows. The purified plasmid (1 µl) was placed in a 0.2 ml-volume PCR tube, and sterile water (4 µl), 5 x BigDye Sequencing Buffer (1 µl), primer ACUN16745(+) (2 µl), and Ready reaction mix (2 µl) were added and mixed. The mixture was PCR amplified at 94°C for 1 minutes with a TaKaRa PCR Thermal Cycler Dice Version III Model TP600 (TaKaRa), followed by 30 cycles of reaction [94°C, 30 seconds → 50°C, 15 seconds → 60°C, 4 minutes], and at 60°C for 3 minutes, and at 4°C for 5 minutes. After the PCR, the total sample amount was placed in a 1.5 ml-volume microtube, and mixed with sterile water (7.25 µl), 3 M sodium acetate (1.5 µl), and 99% ethanol (31.25 µl) for 1 minute with a vortex mixer. Under shaded conditions with an aluminum foil, the mixture was allowed to stand at room temperature for 10 minutes. The mixture was centrifuged at 14,000 rpm (4°C) for 20 minutes. After adding 1 ml of 70% ethanol to the precipitate, the mixture was centrifuged at 14,000 rpm (4°C) for 5 minutes. After the centrifugation, the precipitate was dried with a vacuum drier, and suspended in 20 µl of Hi-Di Formamido. The suspension was boiled for 2 minutes, and immediately allowed to stand on ice to obtain an electrophoresis sample.

The sample was electrophoresed according to the manual of ABI PRISM 3100-Avant Genetic Analyzer. The electrophoresis sample (20 µl) was placed in the wells of a MicroAmp 96-well Reaction Plate, and the plate assembly was installed in an autosampler. The capillary length was 80 cm, POP-4 was used as polymer, and 10 x Genetic Analyzer Buffer with EDTA diluted 10 times with sterile water was used as electrophoresis buffer. After creating a plate record, conditions such as sample name, electrophoresis conditions, and analysis conditions were set. Electrophoresis was performed under the following settings.
Dye Set: Z
Mobility File: DT3100POP4{BDv3}v1.mob,
BioLIMS Project: 3100_Project1
Run Module: Longseq80_POP4DefaultModule
Analysis Module: BC-3100APOP4_80cm_SeqOffFtOff.saz

After the electrophoresis, the analysis data were converted to base sequences according to the manual of ABI PRISM 3100-Avant Genetic Analyzer. The base sequence data were analyzed by using the sequence information analysis software DNASIS Pro ver 2.09.000.001 (HitachiSoft). The result confirmed that the created ZYMV gene fragment was identical to the template.

### 1-2-3: Introduction of ZYMV Gene Fragment to ALSV Vector

Each ZYMV gene fragment with the confirmed sequence was introduced into ALSR2L5R5 after purification and restriction enzyme treatment with BamHI and Xhol, as follows.

TE (72 µl), TE saturated phenol (50 µl), and chloroform (50 µl) were added and mixed with each ZYMV gene fragment (28 µl) for 3 minutes with a vortex mixer. After centrifugation at 14,000 rpm (4°C) for 10 minutes, the aqueous layer (100 µl) was transferred to a new microtube, and 3 M sodium acetate (pH 5.2; 10 µl) and 99% ethanol (250 µl) were added and mixed for 2 minutes with a vortex mixer. The mixture was allowed to stand for 15 minutes in a -80°C freezer, and centrifuged at 14,000 rpm (4°C) for 15 minutes. After removing the supernatant, 70% ethanol (1 ml) was added to the precipitate, and the mixture was centrifuged at 14,000 rpm (4°C) for 5 minutes. The supernatant was removed, and the precipitate was dried with a vacuum drier. The precipitate was suspended in 17 µl of sterile water, and gently mixed with 10 x K Buffer (TaKaRa; 2 µl), BamHI (TaKaRa; 0.5 µl), and Xhol (TaKaRa; 0.5 µl). The mixture was allowed to stand for 12 hours in a 37°C incubator. ALSR2L5R5 was treated with the restriction enzymes in the same manner.

After the restriction enzyme treatment, the ZYMV gene fragments and ALSR2L5R5 were purified according to the method described in Section 1-2-1, using MonoFas DNA purification kit I, and the resulting solutions were obtained as purified Z:P1 gene, purified Z:P3 gene, purified Z:CP200 gene, purified Z:CP100 gene (ZYMV insert DNA), and purified ALSR2L5R5 (vector DNA).

The ZYMV insert DNA and vector DNA were subjected to ligation reaction using a DNA Ligation Kit Ver.2.1 (TaKaRa), as follows. Each insert DNA (4 µl) was placed in a microtube, and the vector DNA (1 µl) and I solution (5 µl) were added to each insert DNA. These were gently mixed, and a ligation reaction was performed in a 16°C water bath for 4 hours. By using each ligation product, plasmids were extracted through transfection of Escherichia coli DH5α, and small-scale culture and boiling as in Section 1-2-1. The extracted plasmids were electrophoresed on 1% agarose to screen for plasmids with possible inserts. The screened plasmids were treated with restriction enzymes BamHI and Xhol to check for the retention of the inserts, as follows. The plasmids (2 µl) were placed in a microtube, and sterile water (6.6 µl), 10 x K Buffer (1 µl), BamHI (0.2 µl), and Xhol (0.2 µl) were added and mixed. The mixture was allowed to stand for 3 hours in a 37°C incubator. This was followed by 1% agarose gel electrophoresis to check for the retention of the inserts in each plasmid.

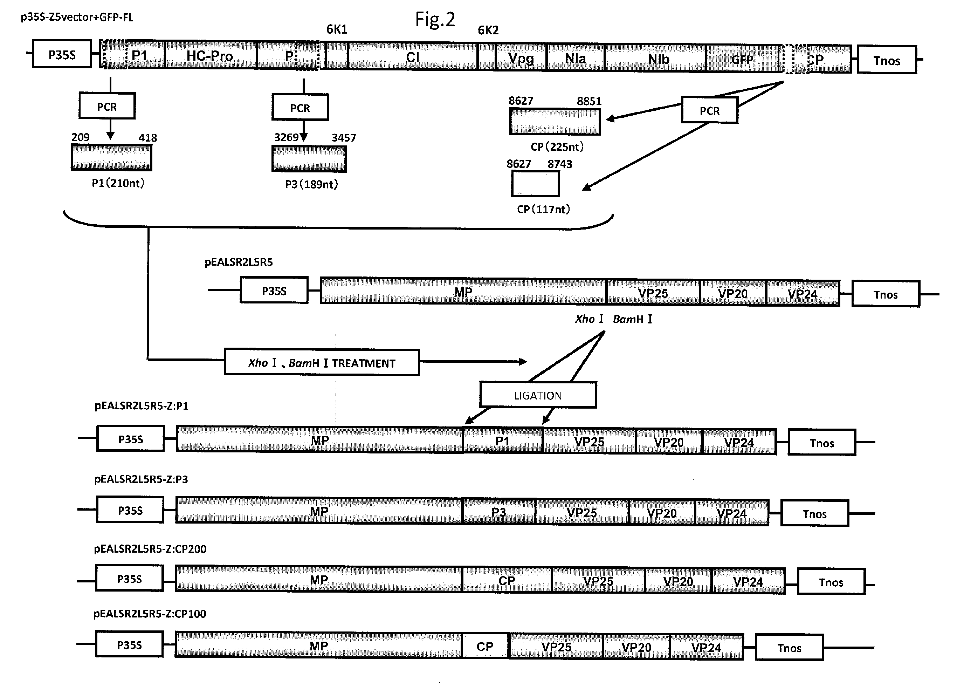

The clones with the retained inserts were grown in large-scale culture, and purified with a QIAGEN Plasmid Midi Kit (QIAGEN) as follows, using a high-speed cooled centrifugal separator himac CR20G (HITACHI) equipped with RPR16 rotor (HITACHI) as a centrifuge. An LB medium (100 ml) was dispensed in a 500 ml-volume Sakaguchi flask, inoculated with colonies with an autoclaved toothpick, and shake cultured at 180 rpm for 14 to 16 hours using a 37°C shaker. The culture medium was transferred to a 50 ml-volume centrifuge tube, and centrifuged at 7,500 rpm (4°C) for 15 minutes. After removing the supernatant, the remaining culture medium was transferred and centrifuged at 7,500 rpm (4°C) for 15 minutes. The supernatant was removed, and the precipitate was suspended in 4 ml of Buffer P1. After adding 4 ml of Buffer P2, the suspension was mixed by inversion, and allowed to stand at room temperature for 4 minutes. Immediately, 4 ml of Buffer P3 was added, and the suspension was mixed by inversion. The mixture was allowed to stand on ice for 20 minutes, and centrifuged at 13,000 rpm (4°C) for 30 minutes. The supernatant was transferred to a new 50 ml-volume centrifuge tube, and centrifuged at 13,000 rpm (4°C) for 15 minutes. The centrifuged supernatant was transferred to the column of QIAGEN-tip 100 equilibrated with 4 ml of Buffer QBT, and the plasmid DNA was adsorbed to the column under free fall conditions. After being washed two times with 10 ml of Buffer QC, the QIAGEN-tip 100 was installed in a new 50ml-volume Sumilon tube, and the DNA was eluted with 5 ml of Buffer QF. Isopropyl alcohol (3.5 ml) was added and mixed with the eluted DNA solution, and the mixture was immediately transferred to a 15 ml-volume COREX tube, and centrifuged at 11,000 rpm (4°C) for 30 minutes. After removing the supernatant, 2 ml of 70% ethanol was added to the precipitate, and the mixture was centrifuged at 11,000 rpm (4°C) for 10 minutes. The supernatant was removed, and the precipitate was dried with a vacuum drier. The precipitate was suspended in 200 µl of TE, and the concentration was adjusted to 1 µg/µl by measuring absorbance (wavelength 260 nm) with a NanoDrop ND-1000 Spectrophotometer. The plasmids were obtained as ALSVR2L5R5-Z:P1, ALSVR2L5R5-Z:P3, ALSVR2L5R5-Z:CP:200, and ALSVR2L5R5-Z:CP:100 (FIG. 2).

### 1-2-4: Inoculation of ALSV Vector and Virus Production

Each purified plasmid (ALSVR2L5R5-Z:P1, ALSVR2L5R5-Z:P3, ALSVR2L5R5-Z:CP200, ALSVR2L5R5-Z:CP100) was mixed with an equal amount of pEALSR1 purified by using the method described in Section 1-2-3 to obtain a DNA solution. The DNA solution was inoculated to the third to sixth true leaves of a quinoa in 7 leaf stage (8 µl per leaf), using the silicon carbide method. After inoculation, upper leaves that showed a chlorosis symptom were sampled, and crushed with 2 volumes of ALSV grinding buffer [0.1 M Tris-HCl (pH 7.8), 0.1 M NaCl,5 mM MgCl₂]. The sample was then inoculated to the third to eighth true leaves of the quinoa in 8 leaf stage by using the silicon carbide method. After inoculation, upper leaves that showed a chlorosis symptom were sampled, and each virus (ALSV-Z:P1, ALSV-Z:P3, ALSV-Z:CP200, ALSV-Z:CP100) was used as the source of inoculum for the later inoculation test. Viruses were also produced for ALSVR2L5R5 in the same manner. The resulting virus wtALSV was used as the source of inoculum for the later inoculation test.

### 1-3: Inoculation to Cucumber, and Primary Inoculation and Secondary Inoculation

### 1-3-1: Primary Inoculation to Cucumber

Primary inoculation to cucumber was performed with the ALSVs (ALSV-Z:P1, ALSV-Z:P3, ALSV-Z:CP200, ALSV-Z:CP100, wtALSV), and ZYMV 2002 by using the following method. Each ALSV infected quinoa leaf was crushed with 2 volumes of ALSV grinding buffer. Each crushed ALSV liquid, and a crushed ZYMV liquid prepared by crushing ZYMV 2002 infected squash leaves with 2 volumes of 0.1 M phosphate buffer (pH 7.0) were inoculated to two cotyledons of a cucumber in the cotyledon developmental stage by using the silicon carbide method. The cucumber was grown in 25°C, long-day conditions.

### 1-3-2: Secondary Inoculation to Cucumber

After the primary inoculation, the cucumber was subjected to secondary inoculation with gold particles coated with p35S-Z5 vector+GFP-FL by using the method described below, using a Helios Gene Gun system (BIO-RAD). Gold particles (BIO-RAD; 10 mg) measuring 1.6 µm in diameter were placed in a 1.5 ml-volume microtube, and 50% glycerol (50 µl) was added and mixed using a vortex mixer. The mixture was sonicated with an ultrasonic bath for 2 minutes, and p35S-Z5 vector+GFP-FL (25 µl) was added while being mixed with a vortex mixer. These were mixed with addition of 1 M CaCl₂ (150 µl) and 0.05 M spermidine (150 µl). The mixture was allowed to stand at room temperature for 15 minutes, and the supernatant was removed with a Pipetman. The precipitate was washed by adding 99% ethanol with care so as not to crumble the precipitate, and the supernatant was removed with a Pipetman. After repeating the washing procedure three times, 99% ethanol (500 µl) was added to the precipitate, and the precipitate was suspended by flicking the microtube with a finger. A dispersion of the gold particles was confirmed. The suspended gold particles were all transferred to a 15ml-volume tube, and 99% ethanol (2.5 ml) was added and mixed. The gold particle suspension so prepared was used for inoculation after coating a gold coating tube according to the Helios Gene Gun quick operation manual, as follows. A suspension loading tube equipped with a syringe was attached to a gold coating tube after drying inside of the tube for 20 minutes with 0.35 to 0.40 LPM (liters per minute) of N₂ gas with a tubing prep-station. The gold particle suspension was then charged into the gold coating tube with the syringe, and allowed to stand for 5 minutes. Then, the suspension loading tube with the syringe was detached, and a syringe-equipped, ethanol removing tube was attached to remove all the ethanol inside the gold coating tube. Immediately after the removal, the gold coating tube was rotated 180° to turn on the rotation switch of the tubing prep-station. Inside of the gold coating tube was dried by aerating the tube with 0.35 to 0.40 LPM of N₂ gas. The dried gold coating tube was then taken out of the tubing prep-station, and cut with a tubing cutter to obtain a sample cartridge. The sample cartridge was attached to the cartridge holder of the Helios Gene Gun, and the first true leaf of the cucumber in 1 leaf stage was inoculated with one shot in 180 PSI (pounds per square inch). This will be referred to as first true leaf secondary inoculation group. The second true leaf in 2 leaf stage, and the third true leaf in 3 leaf stage were also inoculated in the second inoculation in the same manner, and will be referred to as second true leaf secondary inoculation group, and third true leaf secondary inoculation group, respectively. After the secondary inoculation, the cucumber was grown in 25°C, long-day conditions as in the primary inoculation.

The p35S-Z5 vector+GFP-FL, the source of inoculum for the secondary inoculation, was purified by large-scale culture in the same manner as in the method described in Section 1-2-3, except that the LB medium was changed to 2 x YT medium (pH 7.5) [containing bacto tryptone (16 g), bacto yeast extract (10 g),NaCl (5 g), and ampicillin (25 mg/ml; 2 ml) per liter], and was used after being stored at -80°C at a concentration of 400 ng/µl adjusted after measurement with a NanoDrop ND-1000 Spectrophotometer.

### 1-4: Observation of Disease Symptom and Virus Assay

### 1-4-1: Observation of Disease Symptom

Disease symptoms were observed daily after the secondary inoculation, and photographed on day 21 post secondary inoculation, using a digital camera FinePix S1 Pro (FUJIFILM).

### 1-4-2: Observation of GFP Fluorescence

GFP fluorescence was observed on day 7, 14, and 21 post secondary inoculation, and photographed by using a fluorescence microscope system VB series (KEYENCE) and a microscopic digital camera DP70 (OLYMPUS) together.

### 1-4-3: Western Blotting

Two cotyledons were collected from the cucumber on day 7 post primary inoculation, and infection by each primary inoculation virus was confirmed according to the following method. After measuring the weight of the collected two cotyledons, the cotyledons were crushed with 3 volumes of ALSV grinding buffer using a mortar and a pestle, and 100 µl of the crushed plant liquid was transferred to a 1.5 ml-volume microtube. Then, 100 µl of 2 x sample buffer [0.1 M Tris-HCl (pH 8.0), 4% SDS, 30% sucrose, 0.05% BPB, 2% mercaptoethanol] was added and mixed with a vortex mixer for 1 minute. The mixture was boiled for 5 minutes, and immediately allowed to stand on ice for 5 minutes. After centrifugation at 14,000 rpm (4°C) for 5 minutes, the supernatant was obtained as an electrophoresis sample. Ten microliters of the electrophoresis sample was used for electrophoresis (160 V, 20 mA, 90 minutes, room temperature), using a stacking gel (5% acrylamide gel), a running gel (12.5% acrylamide gel), and an electrophoresis buffer [0.6% Tris, 28% glycine, 0.1 % SDS]. After the electrophoresis, the proteins were transferred to a PVDF membrane (MILIPOLE) by semidry electroblotting using a transfer buffer [0.1 M Tris, 0.192 M glycine, 20% methanol]. After the transfer, the PVDF membrane was dipped in a blocking solution [0.02 M Tris-HCl (pH 7.5), 0.5 M NaCl, 2% Tween-20, 2% skim milk, 2% PVP], and allowed to stand for 60 minutes in a 37°C incubator. This was followed by a primary antibody process in a 37°C incubator for 60 minutes with pre-absorbed antibodies against ALSV and ZYMV diluted 25 times with TTBS [0.02 M Tris-HCl (pH 7.5), 0.5 M NaCl, 2% Tween-20]. After the primary antibody process, the PVDF membrane was dipped in a Tupperware container filled with TTBS, and washed for 5 minutes with a seesaw. The washing procedure was repeated two times with a new TTBS. The washed PVDF membrane was then subjected to a secondary antibody process in a 37°C incubator for 60 minutes with an alkaliphosphatase-labeled anti-rabbit immunoglobulin goat serum (Cell Signaling TECHNOLOGY) diluted 2,000 times with TTBS. After the secondary antibody process, the PVDF membrane was washed three times with TTBS, and color development treated with a chromogenic substrate solution [0.2 M Tris-HCl (pH 8.2), 3 mg/ml Fast Red TR salt, 1 mg/ml Naphthol AS-MX phosphate]. After sufficient color development, the PVDF membrane was washed with distilled water to stop the reaction. Note that the pre-absorbed antibodies were produced in advance according to the following method. First, a crushed plant liquid of the test plant crushed with 5 volumes of TTBS was centrifuged at 3,000 rpm (4°C) for 5 minutes, and the supernatant was obtained as a crude juice. Ten microliters of primary antibodies (antiserum against each virus) was placed in a 1.5 ml-volume microtube, and the crude juice (90 µl) was added and mixed. The mixture was allowed to stand at 37°C for 60 minutes, and centrifuged at 14,000 rpm (4°C) for 5 minutes. The supernatant (100 µl) was transferred to a new 1.5 ml-volume microtube. The crushed plant liquid (100 µl) was added and mixed with the supernatant, and the mixture was allowed to stand at 37°C for 60 minutes. After centrifugation at 14,000 rpm (4°C) for 5 minutes, the supernatant (200 µl) was transferred to a new 1.5 ml-volume microtube. The crushed plant liquid (200 µl) was added and mixed with the supernatant, and the mixture was allowed to stand at 37°C for 60 minutes. After centrifugation at 14,000 rpm (4°C) for 5 minutes, the supernatant (400 µl) was transferred to a new 1.5 ml-volume microtube. Then, 400 µl of 100% glycerol was added, and thoroughly mixed with the supernatant with a vortex mixer to obtain pre-absorbed antibodies. The pre-absorbed antibodies were used as appropriate after being stored in a -20°C freezer.

### 1-4-4: ELISA

Accumulation levels of ALSV and ZYMV, and GFP accumulation level were assayed by direct ELISA and indirect ELISA, respectively, according to the following methods.

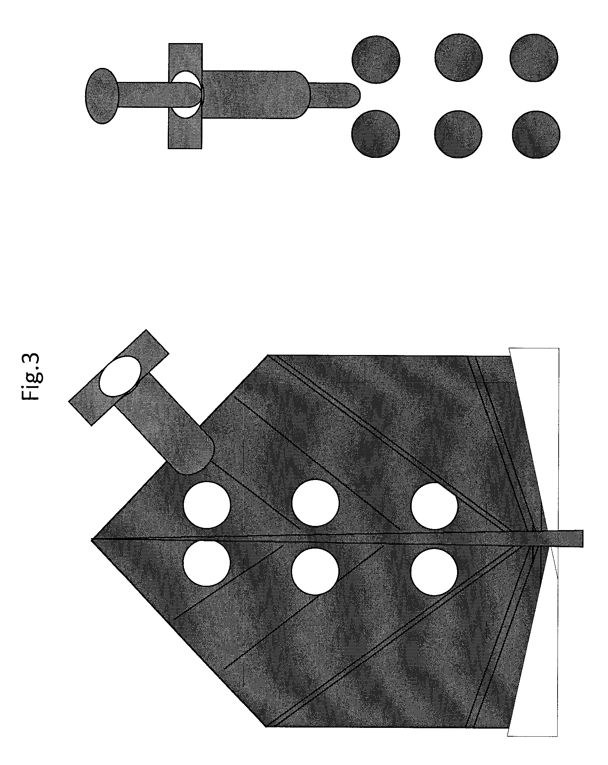

Assay samples were collected on day 21 post secondary inoculation using a cork borer, as follows. The third to sixth true leaves were collected in the first true leaf inoculation group. In the second true leaf inoculation group, the fourth to seventh true leaves were collected, and the fifth to eighth true leaves were collected in the third true leaf inoculation group. Two leaf discs, 1 cm in diameter, were cut out from each of a basal portion, a middle portion, and a peripheral portion of the collected true leaves in areas avoiding thick veins, and a total of six leaf discs were collected from each true leaf (FIG. 3). The six leaf discs were placed in a 2 ml-volume cryopreservation tube (Assist) with a single stainless steel bead SUB-50 (TOMY), and frozen with liquid nitrogen. The leaf samples were then smashed at 2,500 rpm for 30 seconds with a Micro Smash MS100-R (TOMY). Then, 1.8 ml of PBST [137 mM NaCl, 8.1 mM Na₂HPO₄, 2.68 mM KCI, and 1.47 mM KH₂PO₄, 0.05% Tween 20] was added, and mixed at 2,500 rpm for 30 seconds with a Micro Smash MS100-R. The mixture was centrifuged at 14,000 rpm (4°C) for 5 minutes, and the resulting supernatant was obtained as an assay sample.

ALSV was assayed by direct ELISA using the following method. ALSV antibody solution (150 µl) was placed in the wells of an ELISA plate after being diluted 1,000 times with a coating buffer [0.05 M carbonate buffer (pH 9.6)], and allowed to stand in a 37°C incubator for 2 hours with a plate cover placed on top. After discarding the solution, the wells were washed three times with PBST, and 150 µl of a blocking solution [137 mM NaCl, 8.1 mM Na₂HPO₄, 2.68 mM KCI, 1.47 mM KH₂PO₄, 2% skim milk] was placed in each well. The plate was allowed to stand in a 37°C incubator for 1 hour with a plate cover placed on top. After discarding the solution, the wells were washed three times with PBST, and the assay sample was placed in the wells (150 µl each, two wells for each sample), and allowed to stand overnight in a 4°C refrigerator with a plate cover placed on top. After discarding the solution, the wells were washed 10 times with PBST, and 150 µl of an enzyme-labeled ALSV antibody solution was placed in each well after being diluted 1,000 times with PBST. The plate was allowed to stand in a 37°C incubator for 3 hours with a plate cover placed on top. After discarding the solution, the wells were washed 10 times with PBST, and 150 µl of a substrate solution [10% diethanolamine (pH 9.8), 0.67 mg/ml p-nitrophenylphosphoric acid] was added to each well. The plate was allowed to stand at room temperature to allow reaction to proceed until the color developed. After the reaction, the absorbance of each well was measured as the ALSV accumulation level, using a model 550 microplate reader (BIO-RAD).

ZYMV was assayed by direct ELISA using the following method. A ZYMV antibody (Japan Plant Protection Association) solution (150 µl) was placed in the wells of an ELISA plate after being diluted 500 times with a coating buffer, and allowed to stand in a 37°C incubator for 3 hours with a plate cover placed on top. After discarding the solution, the wells were washed three times with PBST, and 150 µl of a blocking solution was placed in each well. The plate was allowed to stand in a 37°C incubator for 1 hour with a plate cover placed on top. After discarding the solution, the wells were washed three times with PBST, and the assay sample was placed in the wells (150 µl each, two wells for each sample). The plate was allowed to stand overnight in a 4°C refrigerator with a plate cover placed on top. After discarding the solution, the wells were washed 10 times with PBST, and 150 µl of a ZYMV antibody (Japan Plant Protection Association) solution was placed in each well after being diluted 1,000 times with PBST. The plate was allowed to stand in a 37°C incubator for 4 hours with a plate cover placed on top. After discarding the solution, the wells were washed 10 times with PBST, and 150 µl of a substrate solution [10% diethanolamine (pH 9.8), 1 mg/ml p-nitrophenylphosphoric acid] was added. The plate was allowed to stand at room temperature to allow reaction to proceed until the color developed. After the reaction, the absorbance of each well was measured as the ZYMV accumulation level, using the model 550 microplate reader.

GFP was assayed by using the following method. The assay sample was placed in the wells of an ELISA plate (150 µl each, two wells for each sample), and allowed to stand in a 37°C incubator for 2 hours with a plate cover placed on top. After discarding the solution, the wells were washed 5 times with PBST, and 150 µl of a blocking solution was placed in each well. The plate was allowed to stand in a 37°C incubator for 1 hour with a plate cover placed on top. After discarding the solution, the wells were washed 5 times with PBST, and 150 µl of GFP antibodies (Clontech) was placed after being diluted 1,000 times with PBST. The plate was allowed to stand overnight in a 37°C refrigerator with a plate cover placed on top. After discarding the solution, the wells were washed 10 times with PBST, and 150 µl of an alkaliphosphatase-labeled anti-rabbit immunoglobulin goat serum was placed in each well after being diluted 2,000 times with PBST. The plate was allowed to stand in a 37°C incubator for 3 hours with a plate cover placed on top. After discarding the solution, the wells were washed 10 times with PBST, and 150 µl of a substrate solution [10% diethanolamine (pH 9.8), 1 mg/ml p-nitrophenylphosphoric acid] was added. The plate was allowed to stand at room temperature to allow for reaction to proceed until the color developed.
After the reaction, the absorbance of each well was measured as the GFP accumulation level, using the model 550 microplate reader.

### 1-4-5: RNA Extraction and RT-PCR

The uppermost developed leaf of the cucumber was collected on day 21 post secondary inoculation, and frozen in a -80°C freezer to obtain an assay sample. The assay sample (0.1 g) was crushed with a mortar and a pestle, and crushed further after adding 1 ml of TriPure Isolation Reagent (Roche). The crushed plant liquid (1 ml) was transferred to a 1.5 ml-volume microtube, and allowed to stand at room temperature for 5 minutes. Then, 200 µl of chloroform was added, and mixed with a vortex mixer for 20 seconds. The mixture was allowed to stand at room temperature for 10 minutes. This was followed by centrifugation at 14,000 rpm (4°C) for 15 minutes, and the aqueous layer (500 µl) was transferred to a new 1.5 ml-volume microtube. Then, water saturated phenol (250 µl) and chloroform (250 µl) were added, and mixed with a vortex mixer for 5 minutes, and the mixture was centrifuged at 14,000 rpm (4°C) for 5 minutes. After the centrifugation, the aqueous layer (500 µl) was transferred to a new microtube, and 420 µl of isopropyl alcohol was added and mixed with a vortex mixer. The mixture was allowed to stand at room temperature for 10 minutes. This was followed by centrifugation at 14,000 rpm (4°C) for 10 minutes. After removing the supernatant, 70% ethanol (1.4 ml) was added, and mixed with a vortex mixer for 5 minutes. The mixture was centrifuged at 14,000 rpm (4°C) for 5 minutes, and the supernatant was removed. The precipitate was suspended in 20 µl of sterile water, and the RNA concentration was adjusted to 500 ng/µl after measurement with a NanoDrop ND-1000 Spectrophotometer to obtain an RNA solution.

The reverse transcription reaction was performed according to the following method. The RNA solution (2 µl), sterile water (6 µl), 5 x RT Buffer (TOYOBO; 4 µl), 2.5 mM dNTP mixture (TaKaRa; 8 µl), 10 µM Oligo(dT)₁₂ primers (1 µl), RNase Inhibitor (Wako; 0.5 µl), and ReverTra Ace (TOYOBO; 0.5 µl) were mixed in a 0.2 ml-volume PCR tube. The mixture was processed at 42°C for 50 minutes, at 95°C for 5 minutes, and then at 4°C for 5 minutes using a TaKaRa PCR Thermal Cycler Dice Version III Model TP600 (TaKaRa). The product was obtained as a RT product.

The following primers were designed to amplify the foreign gene introducing site-containing sequence of ALSV-RNA2 vector in PCR.

Plus-strand primer: 10 µM R2ALS 1363(+) [5'-GCGAGGCACTCCTTA-3'; SEQ ID NO: 8]
Minus-strand primer: 10 µM R2ALS 1511(-) [5'-GCAAGGTGGTCGTGA-3'; SEQ ID NO: 9]

The RT product (1 µl) was transferred to a 0.2 ml-volume PCR tube, and sterile water (4.9 µl), 10 x Ex Taq Buffer (TaKaRa; 1 µl), 2.5 mM dNTP Mixture (TaKaRa; 1 µl), 10 µM R2ALS 1363(+) and 10 µM R2ALS 1511(-) (1 µl each), and TaKaRa Ex Taq™ (TaKaRa; 0.1 µl) were added and mixed. The mixture was PCR amplified at 94°C for 5 minutes, followed by 30 cycles of reaction [94°C, 30 seconds → 58°C, 30 seconds → 72°C, 60 seconds], and at 72°C for 5 minutes, and then 4°C for 5 minutes, using a TaKaRa PCR Thermal Cycler Dice Version III Model TP600 (TaKaRa). The resulting PCR product (2 µl) was mixed with 1 µl of 10 x Loading Buffer (TaKaRa), and electrophoresed on 1% agarose gel to check for the retention of the insert sequence of each ALSV.

### 2: Results

### 2-1: Interference Effect of ZYMV Gene-Introduced ALSV Vector against ZYMV

In order to examine the interference effect of the ZYMV gene fragment-introduced ALSV vector against ZYMV, the cucumbers (Aodai cucumbers) were examined for ZYMV disease symptom, GFP fluorescence, and virus amount after the primary inoculation of wtALSV, and the ALSV with the introduced ZYMV CP gene fragment (ALSV-Z:CP200).

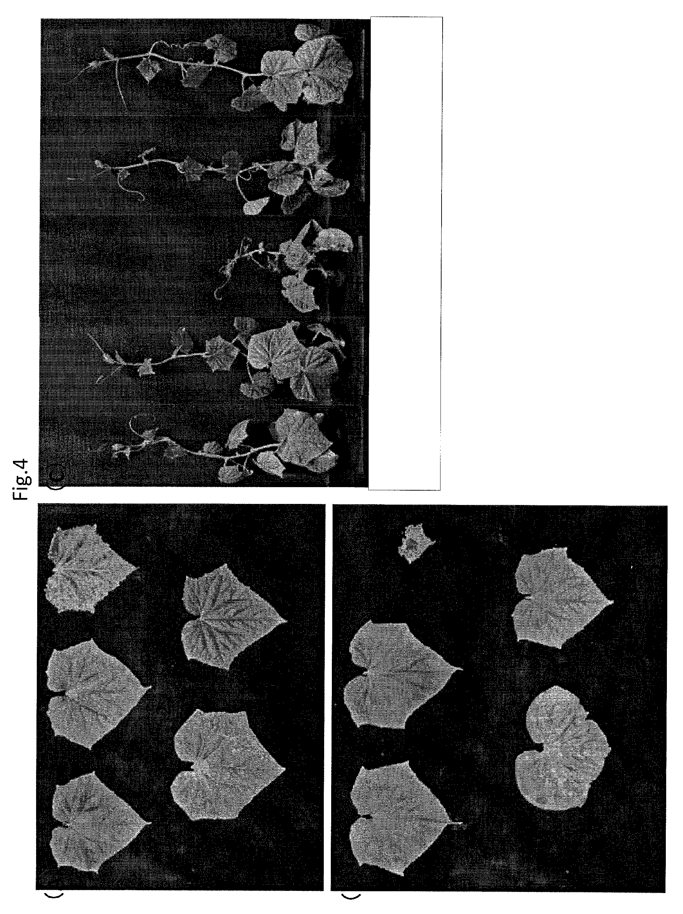

In the test group inoculated with only p35S-Z5 vector+GFP-FL (ZYMV only group), disease symptoms such as chlorotic mottling and interveinal chlorosis were observed in the vicinity of the basal portion of the third true leaf before and after day 12 post inoculation. Mosaic symptoms were observed in the developed fourth true leaf, and severe mosaic symptoms were observed in the fifth and subsequent true leaves, affecting the whole leaf surface (FIG. 4A). Severe mosaic symptoms, and leaf deformation and atrophy were observed in the sixth and subsequent true leaves (FIG. 4B), and the whole plant was dwarfed (FIG. 4C). In the test group inoculated with p35S-Z5 vector+GFP-FL in the secondary inoculation after the primary inoculation of wtALSV (wtALSV+ZYMV group), the disease symptoms in the third to fifth leaves appeared in basically the same patterns as in the ZYMV only group. However, the symptoms were less severe than in the ZYMV only group, and the dwarfing of the whole plant was not observed (FIG. 4). In contrast, in the test group inoculated with p35S-Z5 vector+GFP-FL in the secondary inoculation after the primary inoculation of ALSV-Z:CP200 (ALSV-Z:CP200+ZYMV group), no disease symptoms were observed in any of the upper leaves, as in the non-inoculated group and the test group inoculated with only ALSV (ALSV only group) (FIG. 4).

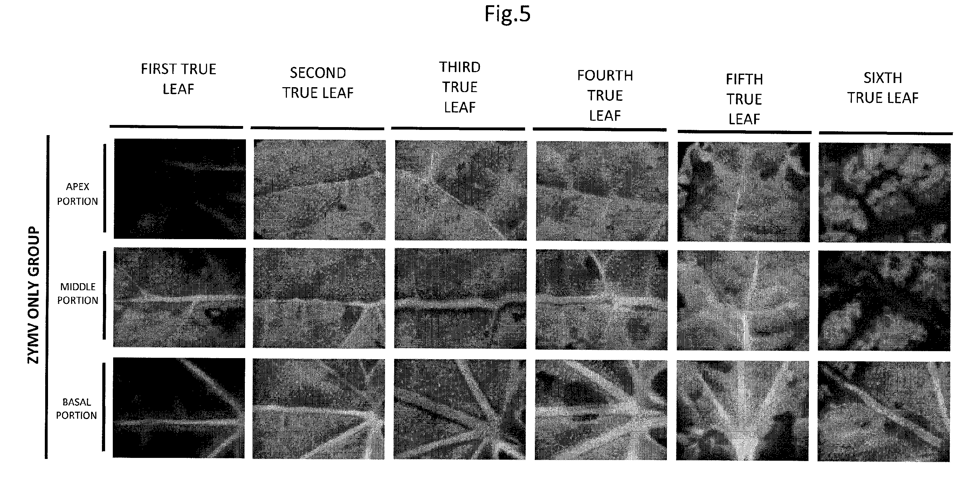

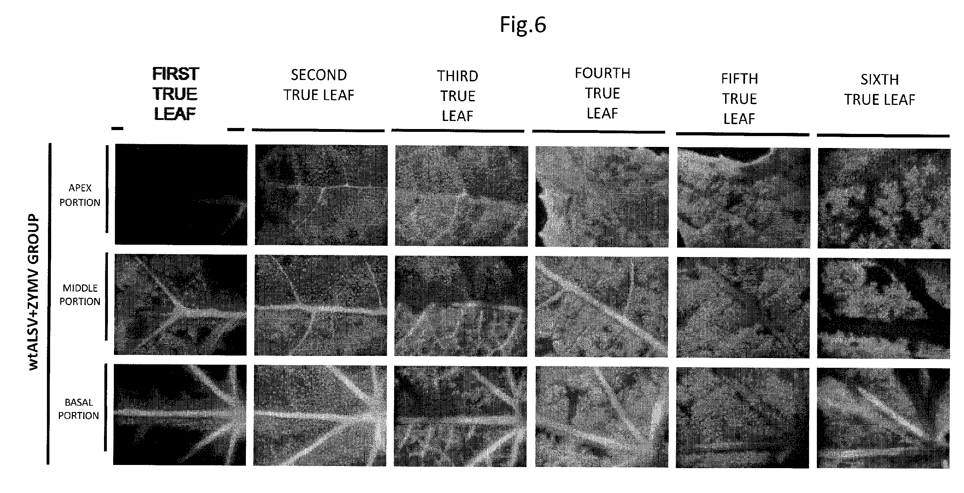

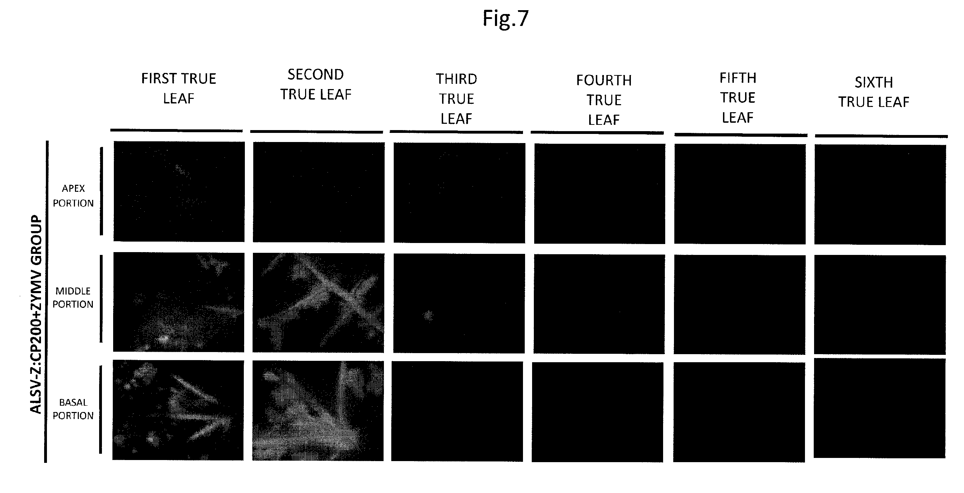

Observation of GFP fluorescence with a fluorescence microscope showed spotty fluorescence in the vicinity of the inoculation site of the first true leaf, and fluorescence on the veins of the first true leaf in the ZYMV only group. In the second true leaf, the vein fluorescence spread over larger areas as the leaf developed. Fluorescence occurred over the whole leaves in the third to fifth true leaves. The sixth true leaf showed fluorescence over the whole leaf, with no fluorescence regions in parts of the leaf (FIG. 5). In the seventh and subsequent leaves, fluorescence and non-fluorescence regions coexisted in a mosaic pattern. The leaves of the wtALSV+ZYMV group showed basically the same fluorescence as in the ZYMV only group (FIG. 6). In the ALSV-Z:CP200+ZYMV group, the first leaf showed spotty fluorescence in the vicinity of the inoculation site, and fluorescence on the veins of the leaf as in the ZYMV only group and the wtALSV+ZYMV group. However, the fluorescence observed on the veins of the second true leaf spread over limited, narrower areas than in the ZYMV group and the wtALSV+ZYMV group. In the third true leaf, fluorescence was observed in only several parts of the leaf, and no fluorescence was observed in the fourth and subsequent leaves (FIG. 7).

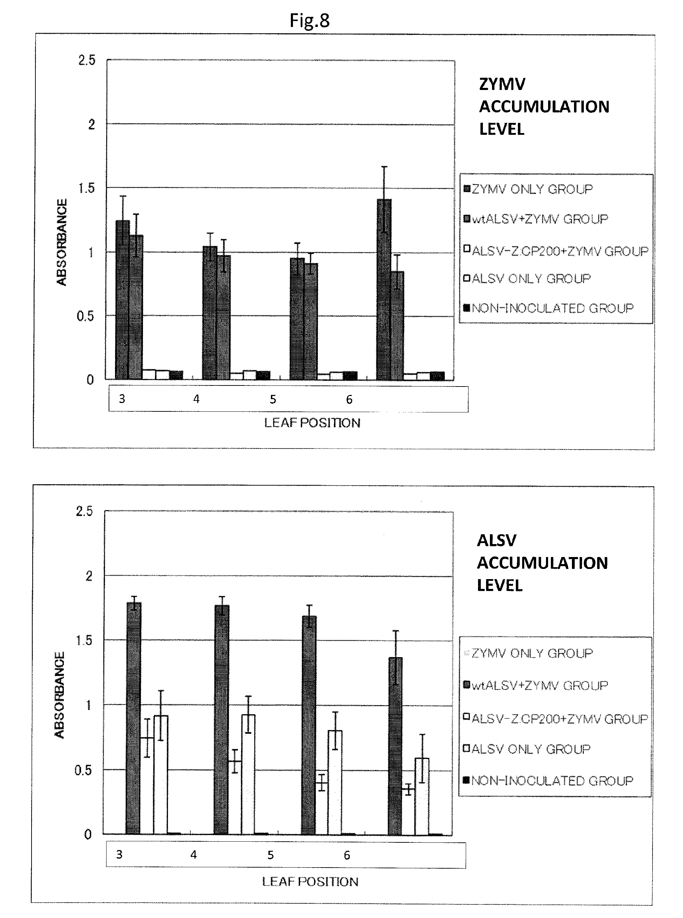

In a ZYMV assay, no ZYMV accumulation was confirmed in the ALSV-Z:CP200+ZYMV group, whereas essentially the same levels of ZYMV accumulation as in the ZYMV only group was observed in the wtALSV+ZYMV group in all of the third to sixth true leaves (FIG. 8). An ALSV assay revealed that the ELISA value was as much as about two times higher in the wtALSV+ZYMV group than in the ALSV only group, and was slightly lower in the ALSV-Z:CP200+ZYMV group than in the ALSV only group (FIG. 8). The ALSV-Z:CP200 used in the primary inoculation was checked for insertion. The retention of the ALSV insert was confirmed in the all cucumber individuals.

The disease symptoms observed in each test group of the second true leaf secondary inoculation group were basically the same as those of the first true leaf secondary inoculation group, except that the disease symptoms were lagged behind by one leaf.

In GFP fluorescence observation, only the second true leaf of the inoculated leaves showed spotty fluorescence in the vicinity of the inoculation site, and fluorescence in parts of the veins, and no fluorescence was observed in the true leaves that developed after the third true leaf in the ALSV-Z:CP200+ZYMV group. In the ZYMV only group and the wtALSV+ZYMV group, GFP fluorescence was observed in the whole leaves as in each test group of the first true leaf secondary inoculation group.

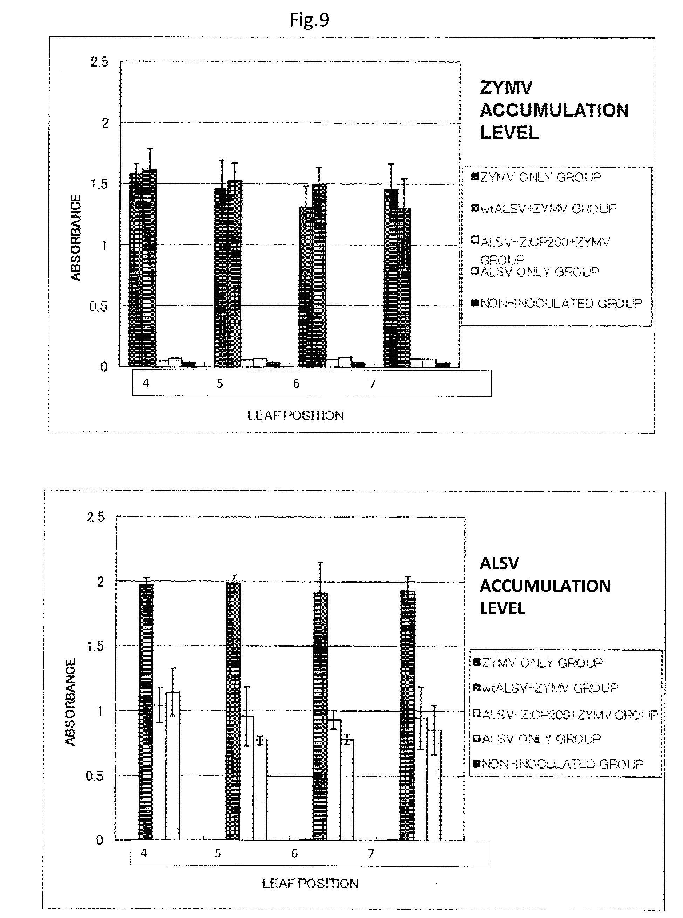

ZYMV accumulation was examined by direct ELISA, and essentially the same ZYMV accumulation was observed in each test group as in the first true leaf secondary inoculation group (FIG. 9). The ALSV-Z:CP200 used for the primary inoculation was checked for insertion. The ALSV insert was retained in the all cucumber individuals.

The patterns of disease symptoms, and the GFP fluorescence distribution in each test group of the third true leaf secondary inoculation group were basically the same as those of the first true leaf secondary inoculation group, except that fluorescence was not observed in any of the true leaves in the ALSV-Z:CP200+ZYMV group, including the inoculated leaves. The disease symptoms and the GFP fluorescence lagged behind by two leaves.

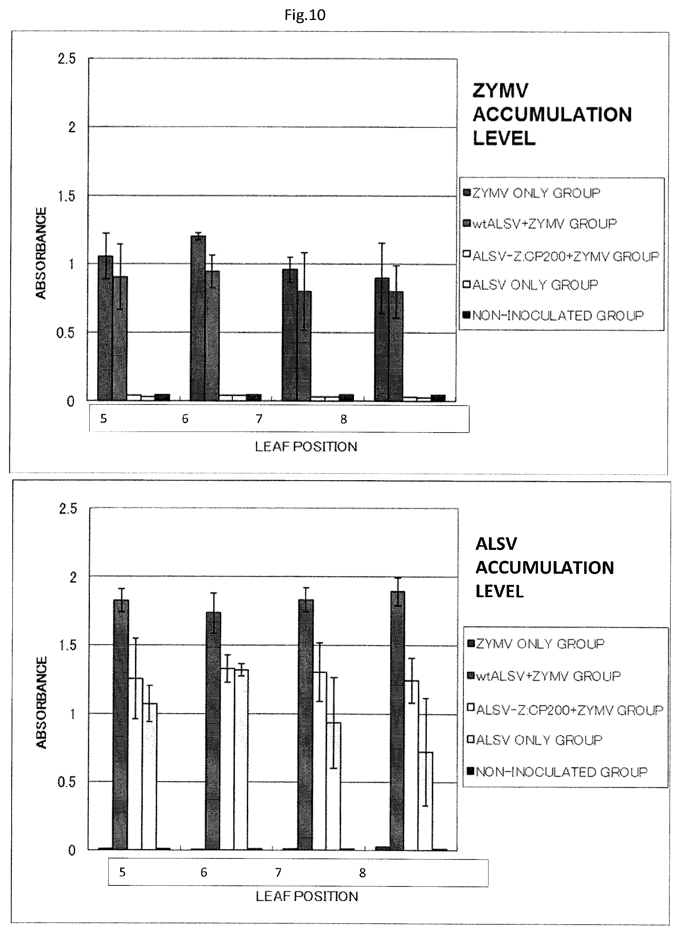

The ZYMV and ALSV assays by direct ELISA revealed that the ALSV accumulation was slightly greater in the ALSV-Z:CP200+ZYMV group than in the ALSV only group, and the results were essentially the same in the other test groups as in the first true leaf secondary inoculation group and the second true leaf secondary inoculation group (FIG. 10). The retention of the ALSV-Z:CP200 insert was also confirmed.

By comparing the ZYMV disease symptoms observed after the p35S-Z5 vector+GFP-FL inoculation in the preliminary test in three cucumber varieties (Aodai cucumber, Suzunari Suyo, Tsubasa), it was found that the symptoms were most severe in the Aodai cucumber, milder in Suzunari Suyo, and almost null in the Tsubasa variety. Accordingly, Aodai was used in all the subsequent tests.

### 2-2: Interference Effect of ALSV Vectors with Introduced Different ZYMV Gene Fragments

In order to compare the interference effects induced by ALSV vectors containing different regions of ZYMV genes, ALSV-Z:P1, ALSV-Z:P3, and ALSV-Z:CP200 were inoculated to the cucumber cotyledons as primary inoculations, and the first true leaf was inoculated with p35S-Z5 vector+GFP-FL in the secondary inoculation.

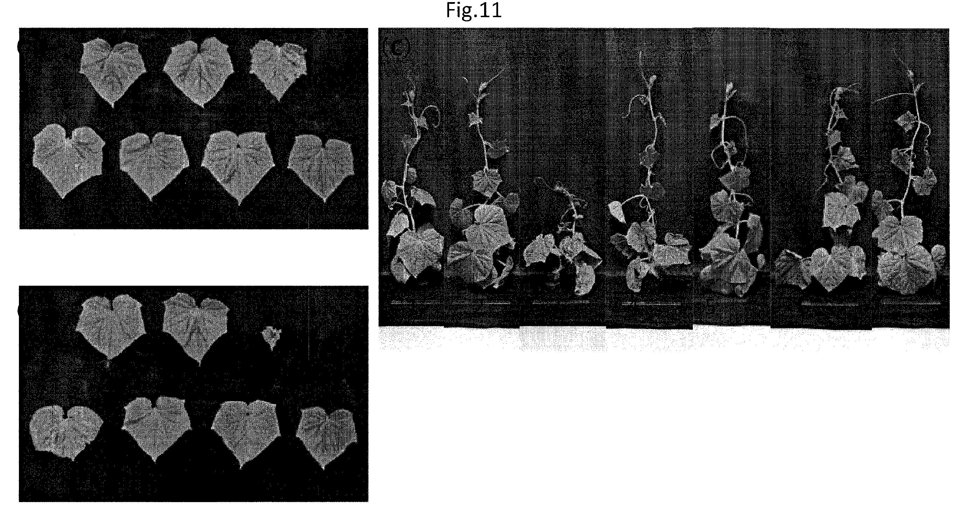

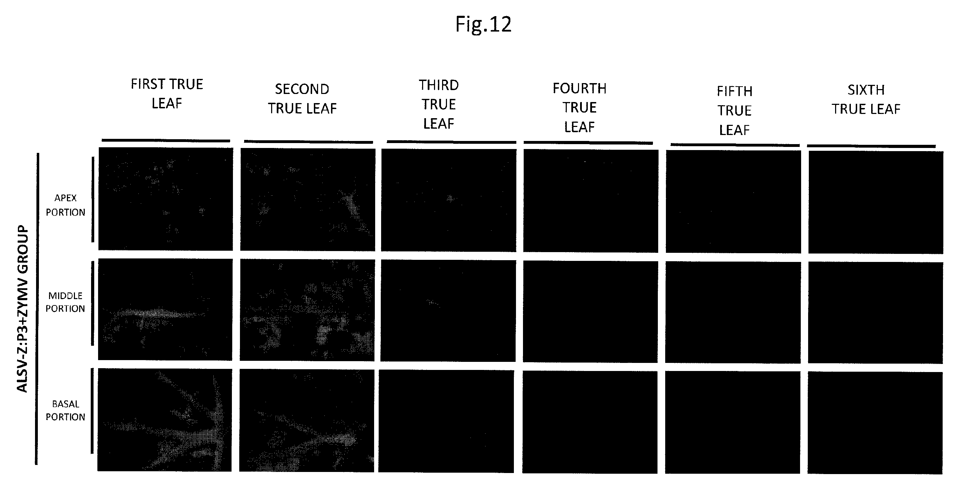

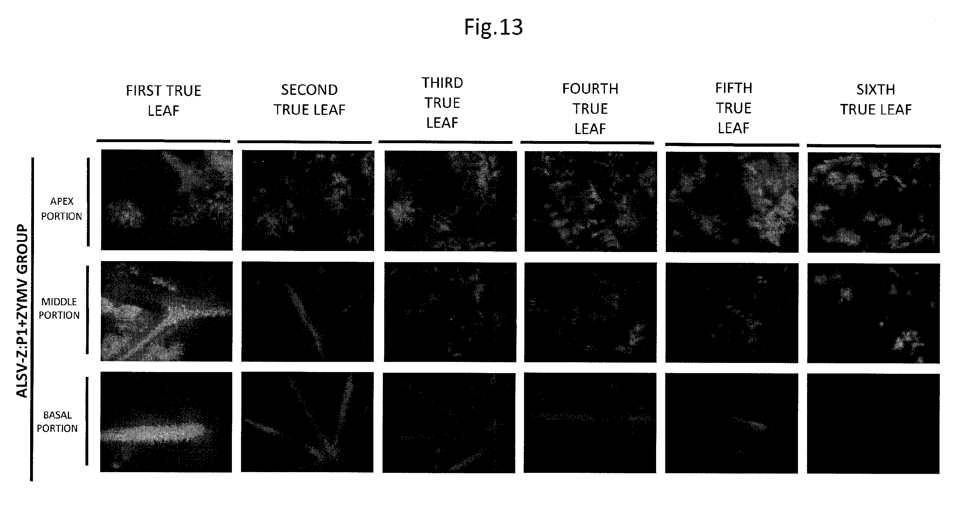

The ALSV-Z:CP200+ZYMV group is as described in Section 2-1. In the test group (ALSV-Z:P3+ZYMV group) inoculated with p35S-Z5 vector+GFP-FL in the secondary inoculation after the primary inoculation of ALSV-Z:P3, no disease symptoms were observed as in the ALSV-Z:CP200+ZYMV group (FIG. 11), and the first true leaf showed spotty fluorescence in the vicinity of the inoculation site, and fluorescence on the veins. The fluorescence observed on the veins of the second true leaf spread over a narrower range. Fluorescence was observed in only several parts of the third and fourth true leaves, and was not observed in the fifth and subsequent true leaves (FIG. 12). In the test group (ALSV-Z:P1+ZYMV group) inoculated with p35S-Z5 vector+GFP-FL in the secondary inoculation after the primary inoculation of ALSV-Z:P1, weak chlorosis symptoms were observed in the leaf peripheral portions in the third true leaf, and the same symptoms were also observed in all the subsequently developed upper leaves. Despite the chlorosis symptoms, the severe mosaic symptoms, and the dwarfing of the leaves and the whole plant observed in the ZYMV only group were not observed (FIG. 11). As for GFP fluorescence, the first true leaf showed spotty fluorescence in the vicinity of the inoculation site, and fluorescence on the veins. The fluorescence on the veins of the second true leaf spread over a narrower range. The third and subsequent true leaves showed fluorescence on the veins, spotty fluorescence in the periphery of the veins, and fluorescence in the periphery and apex portions of the leaves (FIG. 13).

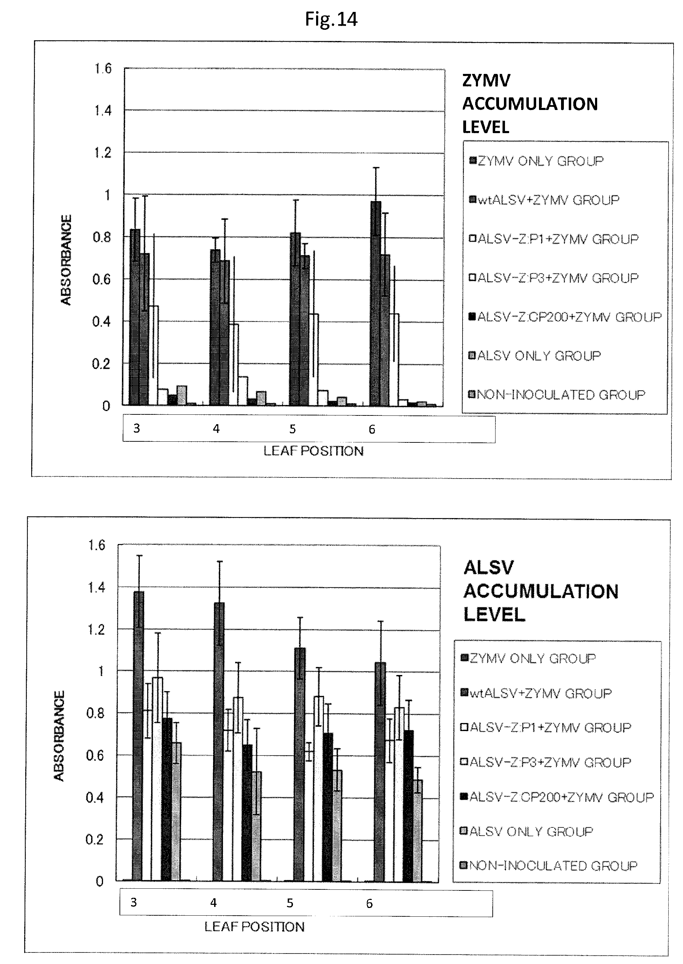

A direct ELISA measurement of ZYMV amount found no ZYMV accumulation in any of the third to sixth true leaves in the ALSV-Z:CP200+ZYMV group and the ALSV-Z:P3+ZYMV group. Accumulation occurred in the ALSV-Z:P1+ZYMV group in about half the amount observed in the ZYMV only group and the wtALSV+ZYMV group (FIG. 14). The ALSV accumulation level was as high as about two times that of the ALSV only group in the wtALSV+ZYMV group, and was slightly higher in the ALSV-Z:P1+ZYMV group, the ALSV-Z:P3+ZYMV group, and the ALSV-Z:CP200+ZYMV group than in the ALSV only group (FIG. 14). The ALSVs (ALSV-Z:P1 , ALSV-Z:P3, ALSV-Z:CP200) with the introduced ZYMV gene fragments used for the primary inoculation were checked for insertion. The ALSV inserts were retained in all the cucumber individuals.

Fragments (180 to 201 nt) of other ZYMV gene regions (HC-Pro, CI, Nla, NIb) were also tested for interference effect. The effects observed for the CP200 fragment and the P3 fragment were also observed for the CI, NIa, and NIb fragments. The HC-Pro fragment showed substantially the same effect as the P1 fragment.

### 2-3: Interference Effect in Different Cucumber Varieties

Two varieties (Suzunari Suyo, and Tsubasa) different from the Aodai cucumber were investigated to find whether the ALSV-Z:CP200 induces the interference effect against ZYMV in these plants.

Only the chlorosis symptoms appeared, and severe mosaic and leaf atrophy, and dwarfing of the plant were not observed in the ZYMV only group and the wtALSV+ZYMV group in Suzunari Suyo. No disease symptoms were observed in the ALSV-Z:CP200+ZYMV group at all. The ZYMV symptoms were not observed in all test groups in Tsubasa.

In the ZYMV only group and the wtALSV+ZYMV group of Suzunari Suyo, the same GFP fluorescence distribution observed in the first true leaf secondary inoculation group of Aodai cucumber was observed in the first to fifth true leaves. The sixth true leaf showed fluorescence, even though some non-fluorescence regions were observed at the basal portion. In the seventh and subsequently developed leaves, spotty fluorescence was observed in many parts of the leaves from the peripheral to the middle portion, and no fluorescence was observed at the basal portion. In the ALSV-Z:CP200+ZYMV group, only weak spotty fluorescence was observed at the periphery of the inoculation site of the first true leaf, and the second true leaf showed only a few fluorescence spots. No fluorescence was observed in the third and subsequent true leaves.

The same GFP fluorescence distribution observed in the Aodai cucumber and Suzunari Suyo was observed in the first to fifth true leaves in the ZYMV only group and the wtALSV+ZYMV group of Tsubasa. The sixth true leaf showed fluorescence, even though some non-fluorescence regions were observed at the basal portion. No fluorescence was observed in the seventh and subsequent true leaves, except for the spotty fluorescence observed along the leaf periphery. In the ALSV-Z:CP200+ZYMV group, only weak spotty fluorescence was observed in the vicinity of the inoculation site of the first true leaf, and the second true leaf showed only a few fluorescence spots. No fluorescence was observed in third and subsequent true leaves.

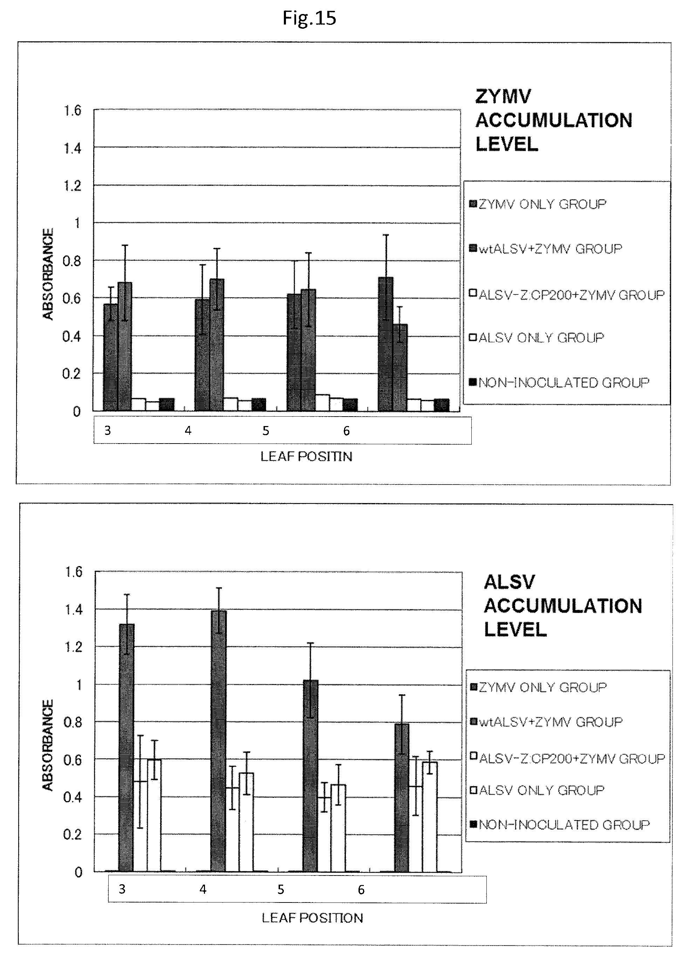

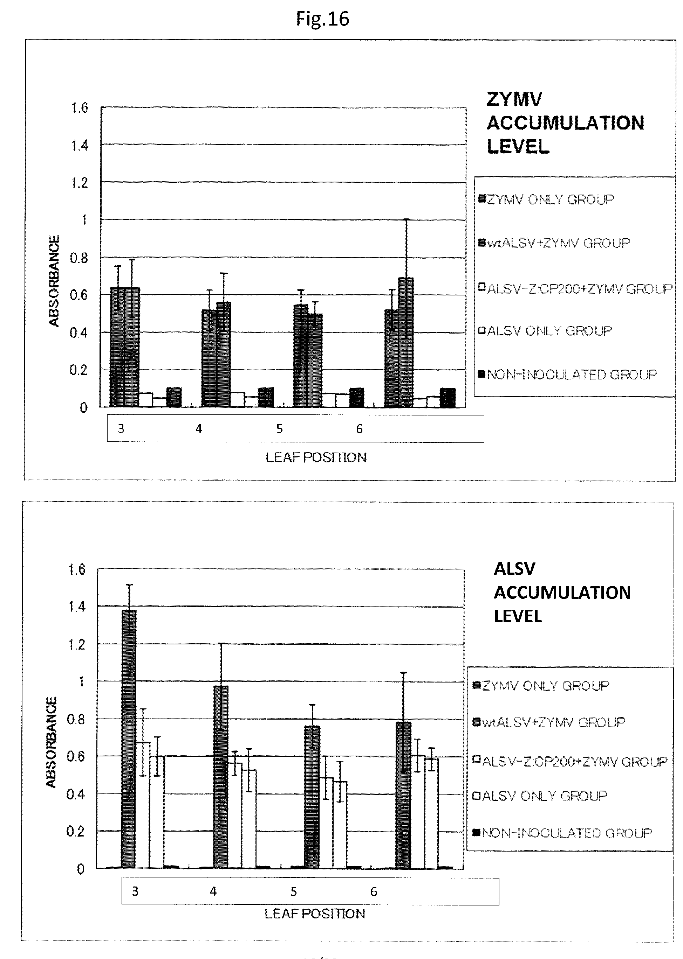

A ZYMV assay by direct ELISA found no ZYMV accumulation in the third to sixth true leaves in the ALSV-Z:CP200+ZYMV groups of Suzunari Suyo and Tsubasa (FIG. 15, FIG. 16). Further, in the Suzunari Suyo and Tsubasa varieties, the ALSV accumulation levels were higher in the wtALSV+ZYMV group than in the ALSV only group, and were slightly lower in the ALSV-Z:CP200+ZYMV group (FIG. 15, FIG. 16). The ALSV-Z:CP200 was checked for insertion. The ALSV insert was retained in the cucumber individuals of the both varieties.

### 2-4: Interference Effect of ALSV Vectors with Introduced ZYMV Gene Fragments of Different Lengths

In order to examine whether the length of the introduced ZYMV gene fragment in the ALSV vector has any effect on the interference effect, the interference effect of the ALSVs (ALSV-Z:CP100, ALSV-Z:CP200) containing the inserted ZYMV-CP gene fragments of about 100 nt and about 200 nt was investigated.

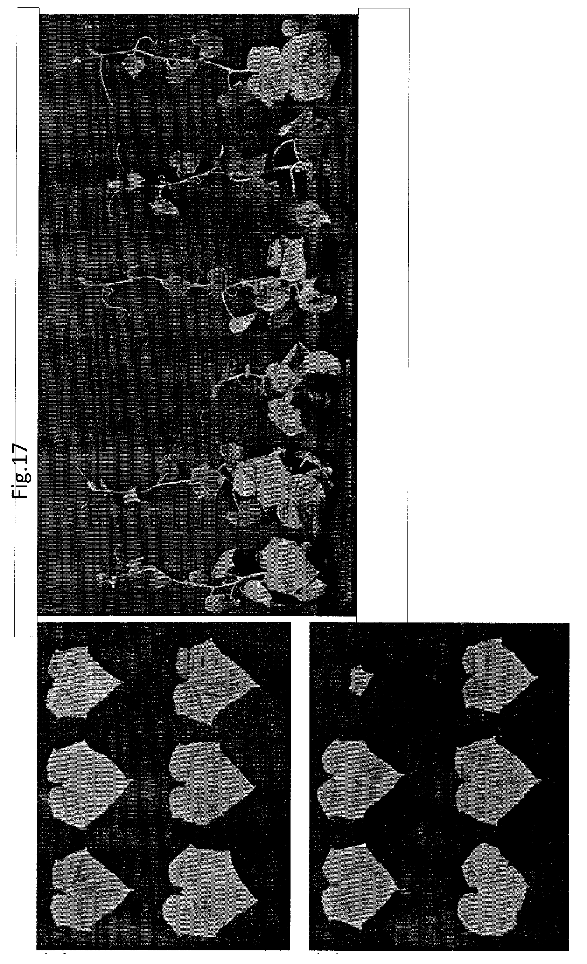

In the ALSV-Z:CP200+ZYMV group, no disease symptoms were observed in any of the upper leaves as in the first true leaf secondary inoculation group in Section 2-1, whereas weak chlorosis symptoms were observed in parts of the leaves in the test group (ALSV-Z:CP100+ZYMV group) inoculated with p35S-Z5 vector+GFP-FL in the secondary inoculation after the primary inoculation of ALSV-Z:CP100 (FIG. 17).

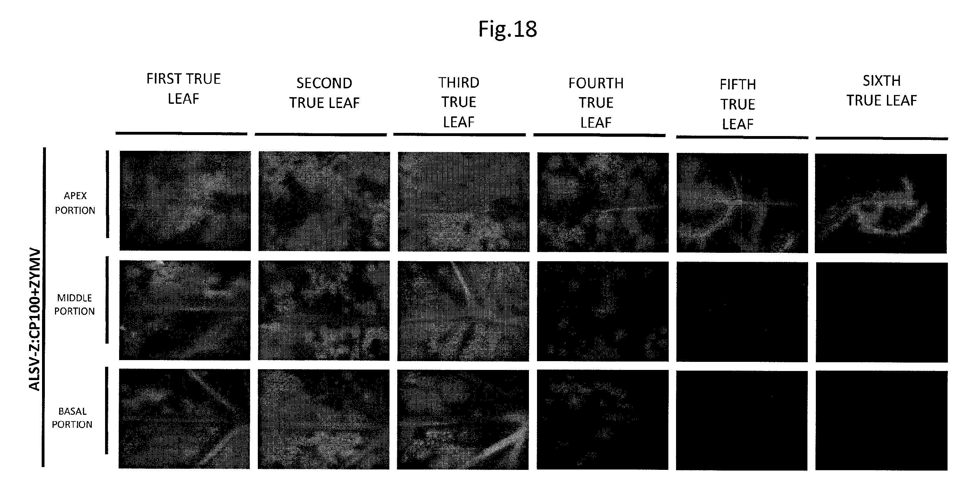

In the ALSV-Z:CP200+ZYMV group, the first true leaf showed spotty GFP fluorescence in the vicinity of the inoculation site, and fluorescence on the veins of the leaf, and the fluorescence on the veins of the second true leaf spread over a narrower range. In the third true leaf, fluorescence was observed in only several parts of the leaf. No fluorescence was observed in the fourth and subsequent true leaves, as in the first true leaf secondary inoculation group in Section 2-1. In the ALSV-Z:CP100+ZYMV group, the first true leaf showed spotty fluorescence in the vicinity of the secondary inoculation site, and fluorescence on the veins of the leaf. Fluorescence was observed on the veins of the second true leaf, and the fluorescence spread as the leaf developed. Fluorescence occurred over the whole leaf in the third true leaf. The fourth true leaf showed spotty fluorescence over the whole leaf, particularly at the peripheral portion of the leaf. The fifth true leaf showed fluorescence on the veins in the peripheral portion of the leaf. Fluorescence was observed only at the leaf apex portion of in the sixth and subsequent true leaves (FIG. 18).

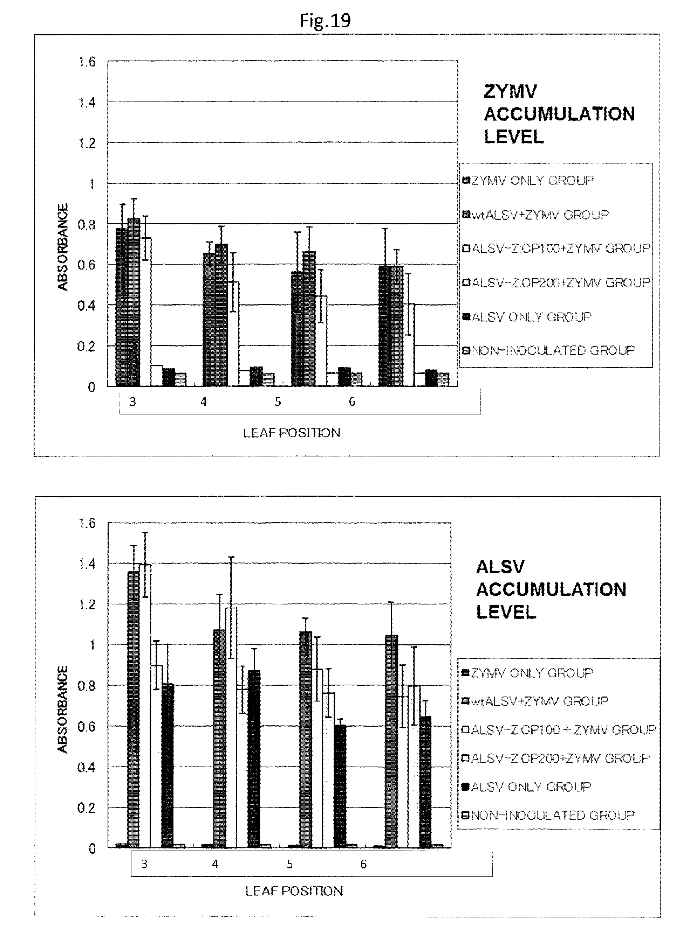

A ZYMV assay by direct ELISA revealed that ZYMV did not accumulate in the third to sixth true leaves in the ALSV-Z:CP200+ZYMV group, but accumulated in the ALSV-Z:CP100+ZYMV group, through the amounts were slightly smaller than in the ZYMV only group and the wtALSV+ZYMY group (FIG. 19). An ALSV assay found increased accumulation levels in the wtALSV+ZYMV group than in the ALSV only group, and about the same accumulation levels in the ALSV-Z:CP200+ZYMV group. The ALSV-Z:CP100+ZYMV group had increased accumulation levels of ALSV in the third to fifth true leaves, and about the same levels of accumulation in the sixth true leaf compared to the ALSV only group (FIG. 19). The ALSVs (ALSV-Z:CP100, ALSV-Z:CP200) with the introduced ZYMV CP gene fragments of different lengths used for the primary inoculation were checked for insertion. The ALSV inserts were retained in all the cucumber individuals.

The interference effects of ALSVs containing the inserted 66 nt and 33 nt ZYMV-CP gene fragments were examined in the same manner. The result confirmed that the interference effect becomes weaker with decreasing sizes of the inserted gene fragments.

### 2-5: Comparison of Interference Effects of ZYMV Gene Fragment-Introduced ALSV and Attenuated ZYMV Strains

Each test group was examined for disease symptom, GFP fluorescence distribution, and virus accumulation to compare the interference effect of the commercially available virus preparation ZYMV 2002, and the interference effects of the three ALSVs containing the introduced ZYMV gene fragments.

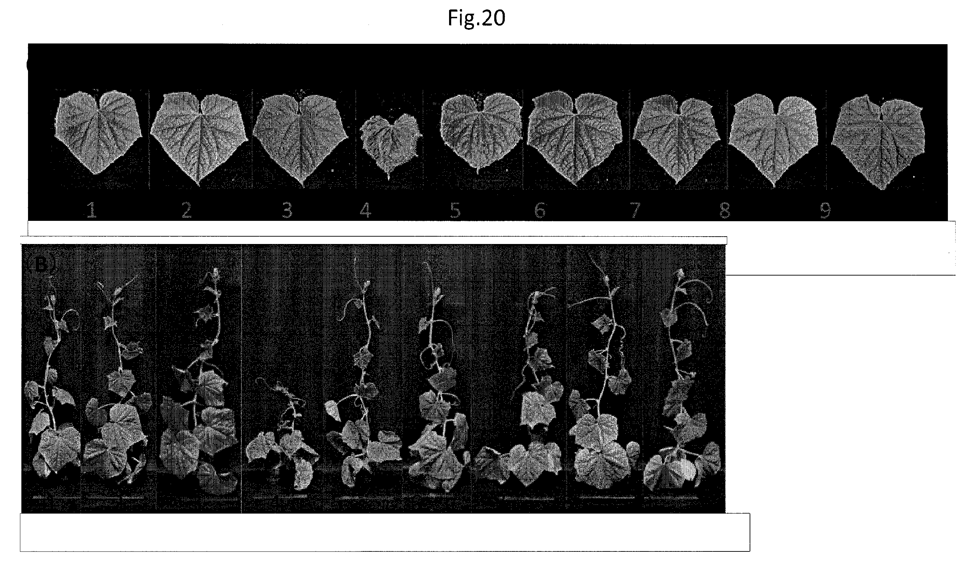

No disease symptoms were observed in the inoculated leaves and the upper leaves in the test group (ZYMV 2002+ZYMV group) inoculated with p35S-Z5 vector+GFP-FL in the secondary inoculation after the primary inoculation of ZYMV 2002 (FIG. 20).

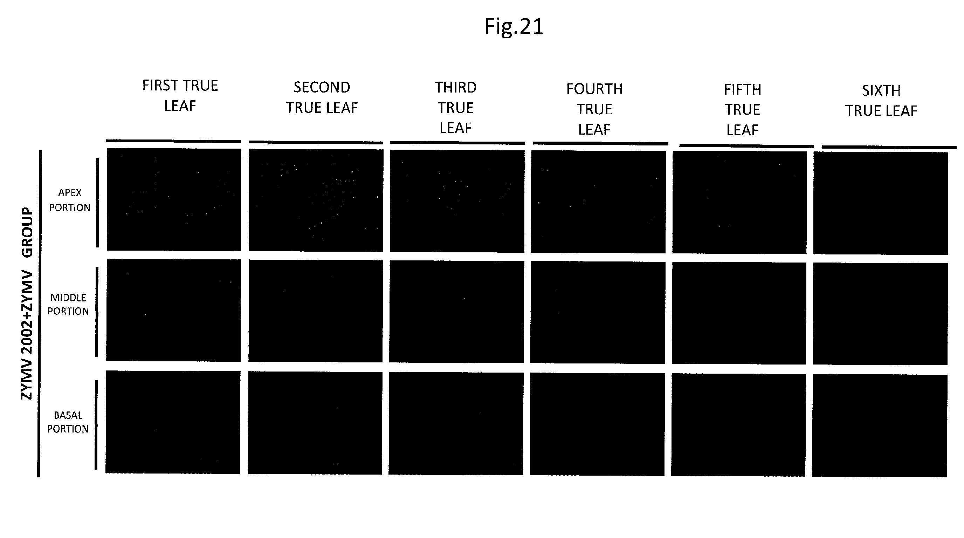

GFP fluorescence was observed in the inoculated leaves and upper leaves in the ALSV-Z:P1+ZYMV group, the ALSV-Z:P3+ZYMV group, and the ALSV-Z:CP200+ZYMV group as in Section 2-2, but not in the ZYMV 2002+ZYMV group (FIG. 21).

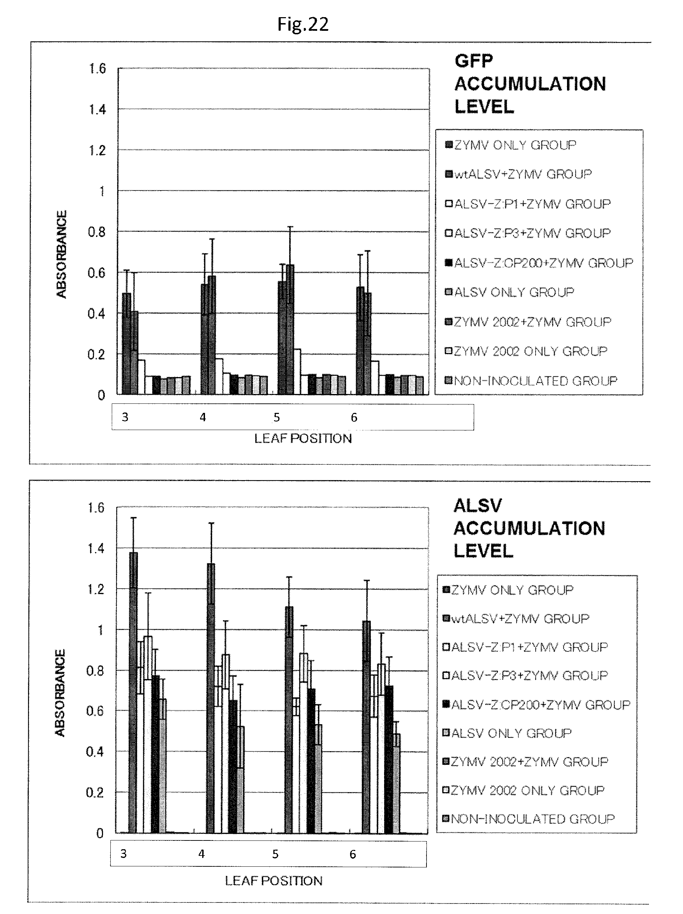

An examination of lethal ZYMV accumulation by indirect ELISA using GFP antibodies revealed GFP accumulation in the ZYMV only group and the wtALSV+ZYMV group, no GFP accumulation in the ALSV-Z:P3+ZYMV group, the ALSV-Z:CP200+ZYMV group, and the ZYMV 2002+ZYMV group, and slight GFP accumulation in the ALSV-Z:P1 +ZYMV group (FIG. 22). The ALSVs (ALSV-Z:P1 , ALSV-Z:P3, ALSV-Z:CP200) with the introduced ZYMV gene fragments used for the primary inoculation were checked for insertion. The ALSV inserts were retained in the all cucumber individuals.

### 3: Discussion

It was envisaged that inoculation of the ZYMV gene fragment-introduced ALSV to cucumbers induces VIGS, and allows the plant to defend against ZYMV. First, in order to examine how the elapsed time after the ALSV vector inoculation affects the induction of the interference effect, infectious clones of the lethal ZYMV strain were inoculated to the first true leaf, the second true leaf, and the third true leaf after 6, 9, and 12 days from the inoculation of the ALSV (ALSV-Z:CP200) with the introduced ZYMV CP gene, and the plant was examined for induction of the interference effect. The result confirmed no ZYMV accumulation in the upper leaves in any one of the three conditions (first true leaf secondary inoculation group, second true leaf secondary inoculation group, and third true leaf secondary inoculation group). GFP fluorescence was observed in the first to third true leaves in the first true leaf secondary inoculation group. In the second true leaf secondary inoculation group, only the inoculated leaves showed GFP fluorescence. No GFP fluorescence was observed even in the inoculated leaves in the third true leaf secondary inoculation group. These results demonstrated that the spread of the ZYMV could be limited by increasing the elapsed time after the ALSV-Z:CP200 inoculation. Taken together with the observation results that the ALSV vector-induced silencing occurred only in regions in the presence of ALSV, and that VIGS was induced always after ALSV proliferation, the results observed in the test groups that had the secondary inoculation to the first and second true leaves can be explained by the proliferation and passage of the ZYMV in the inoculated leaves and upper leaves occurring due to the lack of sufficient time to distribute ALSV. In contrast, the absence of GFP fluorescence in the test group that had the secondary inoculation to the third true leaf was considered to be due to the ALSV being already distributed and having induced VIGS and suppressed ZYMV proliferation. In the test group that had inoculation to the first true leaf, fluorescence was observed in only small parts of the third true leaf. However, a ZYMV assay by direct ELISA found undetectable levels of the viruses, and no disease symptoms were observed in the all test groups. These results show that the interference effect against ZYMV can be induced, and ZYMV proliferation can be suppressed in an ALSV-infected cucumber after at least 6 days from the inoculation of ALSV containing the introduced ZYMV gene fragment.

In order to examine how the different ZYMV gene regions introduced to the ALSV vectors affect the induction of the interference effect, the interference effect was compared among three ZYMV gene-introduced ALSVs (ALSV-Z:P1, ALSV-Z:P3, ALSV-Z:CP200). Weak disease symptoms and GFP fluorescence were observed, and ZYMV accumulated in the ALSV-Z:P1 inoculation group. In contrast, in the ALSV-Z:P3 and ALSV-Z:CP200 inoculated groups, no disease symptoms were observed, and ZYMV accumulation was not confirmed, though some GFP fluorescence was observed. The CI, Nla, and NIb fragments showed results similar to those of the CP200 and P3 fragments.

A comparison test was conducted with ALSV-Z:CP200 to examine whether different cucumber varieties have different effects on the interference effect. The induced vaccine effect was no different among the varieties. It was therefore believed that the variety difference had no effect on the interference effect, and induction of the interference effect by the ALSV vector was considered possible also for varieties other than the foregoing three varieties.

The interference effects of ALSV-Z:CP200 and ALSV-Z:CP100 were compared to examine whether the length of the introduced gene fragment in the ALSV vector has any effect on the induction of the interference effect. ZYMV accumulated, and mild disease symptoms and GFP fluorescence were observed in the test group that had primary inoculation of ALSV-Z:CP100. The induced interference effect was thus believed to be weaker than that of the ALSV-Z:CP200. The result appears to be in agreement with previous reports describing how the extent of silencing differs by the introduction of PDS gene fragments of different sizes, or by the introduction of the same size but different portions of PDS gene fragments to an ALSV vector in inducing silencing of tobacco PDS gene (for example, NPL 4). The weakening of the interference effect with decreasing gene sizes was also confirmed in tests using even smaller inserted genes ALSV-Z:CP66 and ALSV-Z:CP33. This raises the possibility that different interference effects may be induced when the size or site of the introduced gene in the ALSV vector is different in inducing the interference effect against viruses with the ALSV vector. It would therefore be desirable to create ALSVs by introducing more than one virus gene fragment, and compare and examine the interference effect of each ALSV.

Different GFP fluorescence distributions were confirmed in a test comparing the interference effect of commercially available attenuated ZYMV, and the interference effects of the ZYMV gene-introduced ALSVs (ALSV-Z:P1, ALSV-Z:P3, ALSV-Z:CP200). Specifically, no ZYMV GFP fluorescence was observed in the attenuated ZYMV inoculation group at all. This was considered to be due to the different interference effect mechanisms employed by the attenuated virus and the ALSVs. Specifically, the ALSV vector employs the RNA-mediated mechanism by which the interference effect is induced by the silencing of the introduced gene fragment, and silencing is not induced in regions where there is no ALSV distribution. This was believed to be the reason for the proliferation of the lethal strain ZYMV. On the other hand, the interference effect by the attenuated ZYMV occurs among the same viral species, and is protein mediated. This was believed to be the reason for the suppressed proliferation of the lethal ZYMV, and no fluorescence. The virus in the virus infected leaves spread differently between the ALSV vector and the ZYMV. In contrast to the ALSV that spreads from the basal portion of the leaf toward the apex portion, the ZYMV first spreads over the whole veins before seeping out of the veins. This difference in the passage patterns may also represent a factor that affects the interference effect. The increased ALSV accumulation levels in the group that had the secondary inoculation of ZYMV following the ALSV primary inoculation are considered to be due to the synergism of superinfection with ZYMV (for example, NPL 7). This synergism may also be responsible for the weak interference effect of the wtALSV inoculation group, specifically the absence of plant dwarfing.

In sum, introducing the ZYMV gene fragments into the ALSV vector induced the interference effect against ZYMV. However, the extent of the interference effect differed for different types, sizes, and positions of the introduced gene fragments. It would therefore be desirable to create more than one virus gene fragment-introduced ALSV, and compare the effect of each virus for actual applications as an interference effect-inducing vector against viruses.

### Example 2

Interference Effect of ALSV with Introduced Cucumber Mosaic Virus (CMV) Gene Fragment

### 1: Materials and Methods

### 1-1: Experiment Materials

### 1-1-1: Test Plant

A quinoa, a Nicotiana tabacum cv Xanthi nc (tobacco), and a cucumber (Aodai cucumber) were used for the test.

### 1-1-2: Test Virus

Cucumber mosaic virus strain Y (hereinafter, "CMV-Y"; a kind gift from associate professor Hideki Takahashi at Tohoku University, Faculty of Agriculture), and CMV isolate 42CM (hereinafter, "CMV-42CM"; MAFF number: 104087) available from National Institute of Agribiological Science were used as test viruses.

### 1-2: Introduction of CMV Gene Fragment to ALSV Vector

### 1-2-1: Cloning of CMV Gene Fragment

RNA was extracted from CMV-Y-infected tobacco leaves by using the method used in Section 1-4-5 of Example 1. By using the extracted RNA as a template, reverse transcription reaction was performed for the RNA1 (accession number: D12537), RNA2 (accession number:D12538), and RNA3(accession number:D12499) of CMV-Y as follows, using the minus-strand primers CMVY 1a(-), CMVY 2a(-), and CMVY CP(-) (Table 2; SEQ ID NOS: 10 to 15) designed from the known CMV-Y gene sequences.

**Table 2**

| Primer sequences used for cloning of CMV gene fragments | |
|---|---|
| Primer | Sequence (5'-> 3') |
| CMVY 1a(+) | ₉₈ATGGCGACGTCCTCGTTCAA₁₁₇ |
| CMVY 1a(-) | ₈₄₇ACCAACCAGTGGTGTGACGT₈₂₈ |
| CMVY 2a(+) | ₁₀₆GCATTCTCACTAGCCAATCT₁₂₅ |
| CMVY 2a(-) | ₇₆₈ATCATCAGCGAAAGTCCTCT₇₄₉ |
| CMVY CP(+) | ₁₂₆₉TCTGAATCAACCAGTGCTGG₁₂₈₈ |
| CMVY CP(-) | ₁₈₂₆TGAATACACGAGGACGGCGT₁₈₀₇ |

An RNA solution (2 µl), sterile water (6 µl), 5 x RT Buffer (4 µl), 2.5 mM dNTP mixture (TaKaRa; 8 µl), 10 µM minus-strand primers (1 µl), RNase Inhibitor (0.5 µl), and ReverTra Ace (0.5 µl) were mixed in a 0.2 ml-volume PCR tube. The mixture was processed at 42°C for 50 minutes, and 95°C for 5 minutes, and then at 4°C for 5 minutes using a TaKaRa PCR Thermal Cycler Dice Version III Model TP600. The resulting product was obtained as a RT product.

By using the RT product as a template, each gene fragment was PCR amplified by using the same method used in Section 1-2-1 of Example 1. The following primer sets were used (Table 2).
CMVY 1 a(+) and CMVY 1 a(-) for 1 a gene (750 nt)
CMVY 2a(+) and CMVY 2a(-) for 2a gene (663 nt)
CMVY CP(+) and CMVY CP(-) for CP gene (558 nt)

The size of each PCR product was confirmed, and the PCR products were purified with MonoFas DNA purification kit I in the same manner as in Section 1-2-1 of Example 1. The purified gene fragments were obtained as C:1a gene fragment, C:2a gene fragment, and C:CP gene fragment.

TA cloning of the purified three gene fragments using a pGEM-T Easy Vector System, transfection of Escherichia coli DH5α with the ligated products, extraction of plasmids by small-scale culture and boiling, screening of the extracted plasmids by electrophoresis with 1% agarose gel, and confirmation of the retention of the inserts by restriction enzyme treatment with EcoRI were performed by using the same methods used in Section 1-2-1 of Example 1.

### 1-2-2: CMV Gene Fragment Sequence

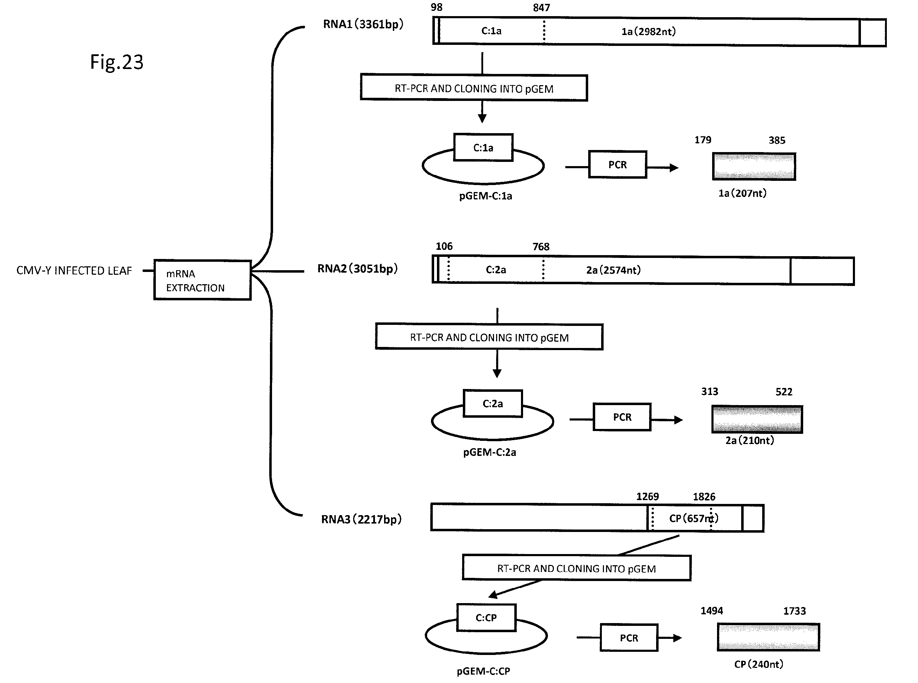

The plasmids produced by TA cloning were purified, and the sequences were analyzed by using the same methods used in Section 1-2-2 of Example 1. The plasmids with the confirmed sequences were obtained as pGEM-C:1a, pGEM-C:2a, and pGEM-C:CP (FIG. 23).

### 1-2-3: Introduction of CMV Gene Fragment into ALSV Vector

PCR was performed in the same manner as in Section 1-2-1 of Example 1, using pGEM-C:1a, pGEM-C:2a, and pGEM-C:CP as templates. The following primer sets were used (Table 3; SEQ ID NOS: 16 to 21).
pGEM-C:1a: CMV-Y 1a Xho(+) and CMV-Y 1a Bam(-)
pGEM-C:2a: CMV-Y 2a Xho(+) and CMV-Y 2a Bam(-)
pGEM-C:CP: CMV-Y CP Xho(+) and CMV-Y CP Bam(-)

The restriction enzyme treatment of the PCR products using BamHI and Xhol, and purification using a MonoFas DNA purification kit I were performed by using the methods used in1-2-3 of Example 1. Each CMV insert DNA and vector DNA were ligated by using a DNA Ligation Kit Ver.2.1, and Escherichia coli DH5α was transfected. The cells were grown in small-scale culture, and the plasmids were extracted by boiling.

**Table 3**

| Primer sequences used for introduction of CMV gene fragment into ALSV vector | |
|---|---|
| Primer | Sequence (5'-> 3') |
| CMV-Y 1a Xho(+) | AAGACAACTCATGAGCAACTCGAG₁₇₉GAGCAA₁₈₄ |
| CMV-Y 1a Bam(-) | CGCGGATCC₃₈₅TAGACAATCGAGAGTTCCACA₃₆₅ |
| CMV-Y 2a Xho(+) | CCGCTCGAG₃₁₃GATGAGTTTGTAACTTATGGT₃₃₃ |
| CMV-Y 2a Bam(-) | CGCGGATCC₅₂₂TTCAAAACACTTCATGGTTCG₅₀₂ |
| CMV-Y CP Xho(+) | CCGCTCGAG₁₄₉₄AAAATAGACCGTGGGTCTTAT₁₅₁₄ |
| CMV-Y CP Bam(-) | CGCGGATCC₁₇₃₃AGATGCAGCATACTGATAAAC₁₇₁₃ |

Restriction enzyme recognition sequences are underlined.

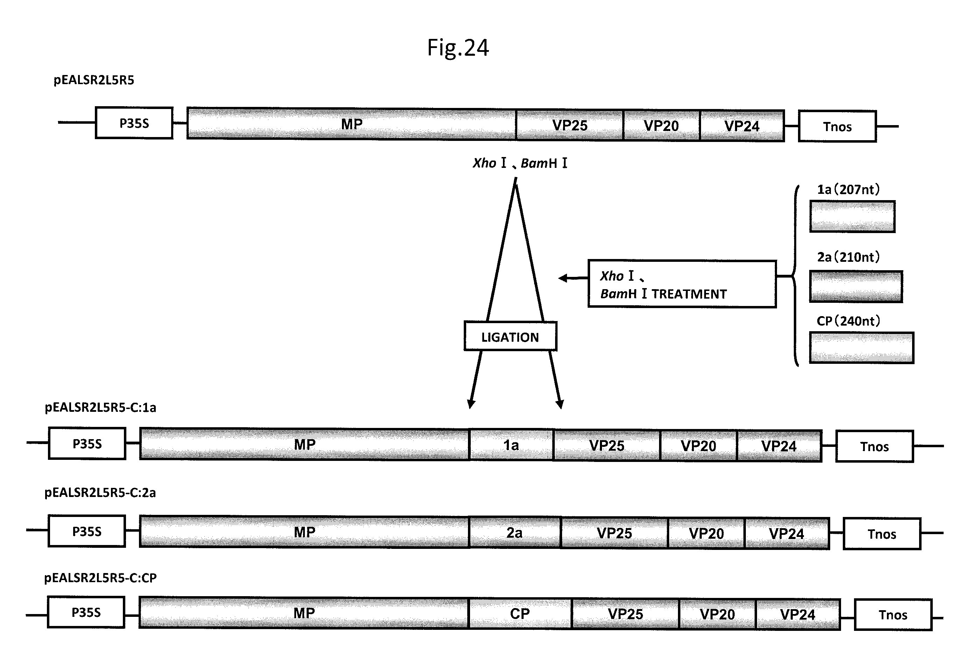

The clone carrying each CMV insert DNA was adjusted to a concentration of 1 µg/µl, and the plasmids were obtained as ALSVR2L5R5-C:1a:, ALSVR2L5R5-C:2a, and ALSVR2L5R5-C:CP (FIG. 24).

### 1-2-4: Inoculation of ALSV Vector and Virus Production

Viruses were produced from the purified plasmids (ALSVR2L5R5-C:1a, ALSVR2L5R5-C:2a, ALSVR2L5R5-C:CP) in the same manner as in Section 1-2-4 of Example 1. The viruses obtained by inoculation of each plasmid were designated as ALSV-C:1a, ALSV-C:2a, and ALSV-C:CP, and were used as the source of inoculum for the later tests.

### 1-3: Inoculation to Test Plant, and Primary Inoculation and Secondary Inoculation

### 1-3-1: Primary Inoculation

Primary inoculation to the tobacco was performed as follows. Quinoa leaves infected with wtALSV, ALSV-C:1a, ALSV-C:2a, and ALSV-C:CP were crushed with 2 volumes of ALSV grinding buffer. The resulting each crushed ALSV liquid was inoculated to the third to fifth true leaves of tobacco in 5 leaf stage, using the silicon carbide method. The tobacco was grown in 25°C, long-day conditions.

Primary inoculation to the cucumber was performed in the same manner as in1-3-1 of Example 1, and the plant was grown in 25°C, long-day conditions.

### 1-3-2: Secondary Inoculation

Secondary inoculation to the tobacco was performed with CMV-Y, as follows. A CMV-Y-infected tobacco leaf was crushed with 2 volumes of 0.1 M phosphate buffer (pH 7.0), and inoculated to the seventh to ninth true leaves as secondary inoculation, using the silicon carbide method. After the secondary inoculation, the tobacco was grown in 25°C, long-day conditions, as after the primary inoculation.

Secondary inoculation to the cucumber was performed with CMV-42CM, as follows. A CMV-42CM-infected tobacco leaf was crushed with 2 volumes of 0.1 M phosphate buffer (pH 7.0), and inoculated to the first true leaf as secondary inoculation, using the silicon carbide method. After the secondary inoculation, the cucumber was grown in 25°C, long-day conditions, as after the primary inoculation.

The CMV-Y and the CMV-42CM used for the secondary inoculation were prepared by inoculating a tobacco with the virus using a 0.1 M phosphate buffer (pH 7.0), and collecting the upper leaves of the plant that showed disease symptoms, and were used after being cryopreserved at -80°C.

### 1-4: Observation of Disease Symptoms, and Virus Assay

### 1-4-1: Observation of Disease Symptoms

Disease symptoms were observed daily after the secondary inoculation. The observed disease symptoms were photographed with a digital camera FinePix S1 Pro on day 9 post secondary inoculation for the tobacco, and on day 18 post secondary inoculation for the cucumber.

### 1-4-2: Western Blotting

The third to fifth true leaves of the tobacco were collected, and two cotyledons were collected from the cucumber on day 7 post primary inoculation. These were used for western blotting performed in the same manner as in 1-4-3 of Example 1 to confirm ALSV infection following the primary inoculation.

### 1-4-3: ELISA

Accumulation levels of CMV-Y and ALSV in the tobacco were assayed by direct ELISA using assay samples. The assay samples were prepared as follows. The twelfth to fifteenth true leaves were collected on day 9 post secondary inoculation, and crushed with 20 volumes of PBST, using a mortar and a pestle. The crushed plant liquid (1 ml) was transferred to a 1.5 ml-volume microtube, and centrifuged at 14,000 rpm (4°C) for 5 minutes. The resulting supernatant was obtained as an assay sample. The assay sample produced as above was then used for the virus assay, which was performed by direct ELISA as in 1-4-4 of Example 1.

Accumulation levels of CMV-42CM and ALSV in the cucumber were assayed by direct ELISA using assay samples as in 1-4-4 of Example 1. The assay samples were prepared by collecting six leaf discs from each of the third to sixth true leaves on day 18 post secondary inoculation in the same manner as in 1-4-4 of Example 1.

CMV antibodies (Japan Plant Protection Association), and enzyme-labeled CMV antibodies (Japan Plant Protection Association) were used for the CMV-Y and CMV-42CM assays by direct ELISA.

### 1-4-4: RNA Extraction and RT-PCR

The uppermost leaf was collected on day 9 post secondary inoculation in the tobacco, and on day 18 post secondary inoculation in the cucumber. These were frozen at -80°C, and used as assay samples. RNA extraction, reverse transcription reaction, and PCR were performed by using the same methods used in Section 1-4-5 of Example 1 to confirm the retention of the ALSV insert sequence.

### 2: Results

### 2-1: Interference Effect of ALSV Vector Containing CMV-Y Gene Fragment in Tobacco

In order to examine whether the interference effect against CMV-Y could be induced in tobacco through inoculation of the ALSV vectors containing the CMV-Y gene fragments, CMV-Y was inoculated in the secondary inoculation after the primary inoculation of a tobacco with wtALSV and ALSVs (ALSV-C:1a, ALSV-C:2a, ALSV-C:CP) containing the introduced CMV-Y gene fragments. These were then tested for induction of the interference effect against CMV-Y.

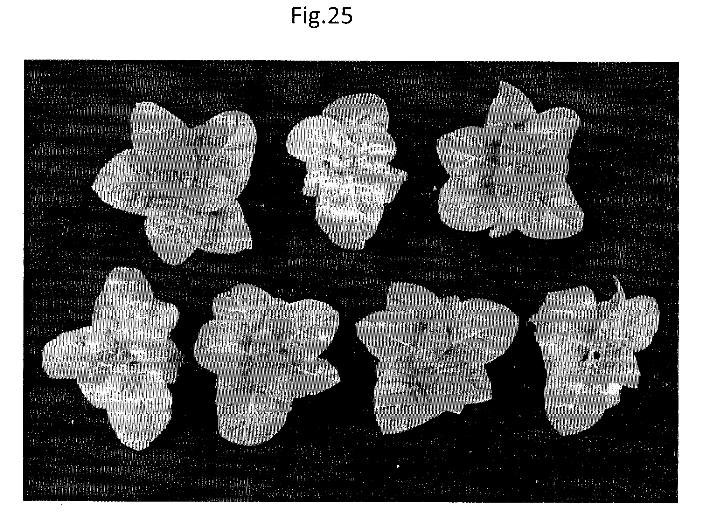

Yellow spots were observed in the inoculated leaves of all individuals after about 3 days from the secondary inoculation, and severe mosaic symptoms appeared on the upper leaves after about 7 days in the test group that did not have the primary inoculation of ALSV but had the secondary inoculation of only CMV-Y (CMV-Y only group), and in the test group that had the secondary inoculation of CMV-Y after the primary inoculation of wtALSV (wtALSV+CMV-Y group). In contrast, no disease symptoms were observed in any of the individuals in the test group that had the secondary inoculation of CMV-Y after the primary inoculation of ALSV-C:2a (ALSV-C:2a+CMV-Y group). Three of the six individuals showed weak mosaic symptoms in the upper leaves, and the remaining three did not show any disease symptom in the test group that had the secondary inoculation of CMV-Y after the primary inoculation of ALSV-C:1a (ALSV-C:1a+CMV-Y group). On the other hand, weak mosaic symptoms were observed in the upper leaves in the all six individuals in the test group that had the secondary inoculation of CMV-Y after the primary inoculation of ALSV-C:CP (ALSV-C:CP+CMV-Y group) (FIG. 25, Table 4).

**Table 4**

| Interference effect against CMV-Y in primary virus inoculation test group (9 dpi) | | | |
|---|---|---|---|
| Primary inoculation virus | Secondary inoculation virus | Symptom developed individuals/CMV-Y inoculated individuals | Severity of disease symptoms |
| No inoculation | CMV-Y | 6/6 | +++ |
| wtALSV | CMV-Y | 5/5 | +++ |
| ALSV-C:1a | CMV-Y | 3/6 | + or - |
| ALSV-C:2a | CMV-Y | 0/6 | - |
| ALSV-C:CP | CMV-Y | 6/6 | + |

| | | | |
|---|---|---|---|
| ^{*} +++: Severe symptoms; +: Mild symptoms; -: No symptoms | | | |

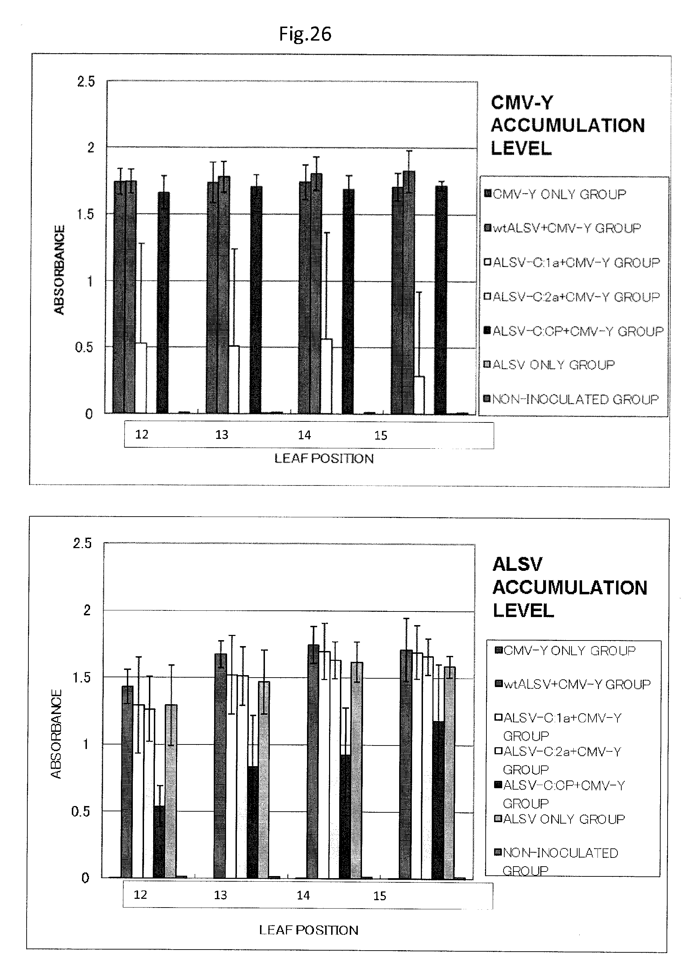

No CMV-Y accumulation was confirmed in the ALSV-C:2a+CMV-Y group, whereas the ALSV-C:CP+CMV-Y group had the same levels of accumulation observed in the CMV-Y only group and the wtALSV+CMV-Y group. The accumulation in the ALSV-C:1a+CMV-Y group was about 1/3 of that observed in the CMV-Y only group and the wtALSV+CMV-Y group (FIG. 26). An ALSV assay confirmed the same levels of accumulation in the ALSV-C:1a+CMV-Y group and the ALSV-C:2a+CMV-Y group as in the ALSV only group, and slightly increased accumulation levels in the wtALSV+CMV-Y group compared to the ALSV only group. On the other hand, the accumulation in the ALSV-C:CP+CMV-Y group was less than by about half to 1/4 of the amount observed in the ALSV only group (FIG. 26). The ALSVs (ALSV-C:1 a , ALSV-C:2a, ALSV-C:CP) containing the introduced CMV-Y gene fragments used for the primary inoculation were checked for insertion. The ALSV inserts were retained in the all cucumber individuals.

### 2-2: Interference Effect of ALSV Vectors with CMV-Y Gene Fragments in Cucumber

The interference effect against CMV-42CM was examined in a cucumber that had primary inoculation of wtALSV and ALSVs (ALSV-C:1a, ALSV-C:2a, ALSV-C:CP) containing the introduced CMV-Y gene fragments.

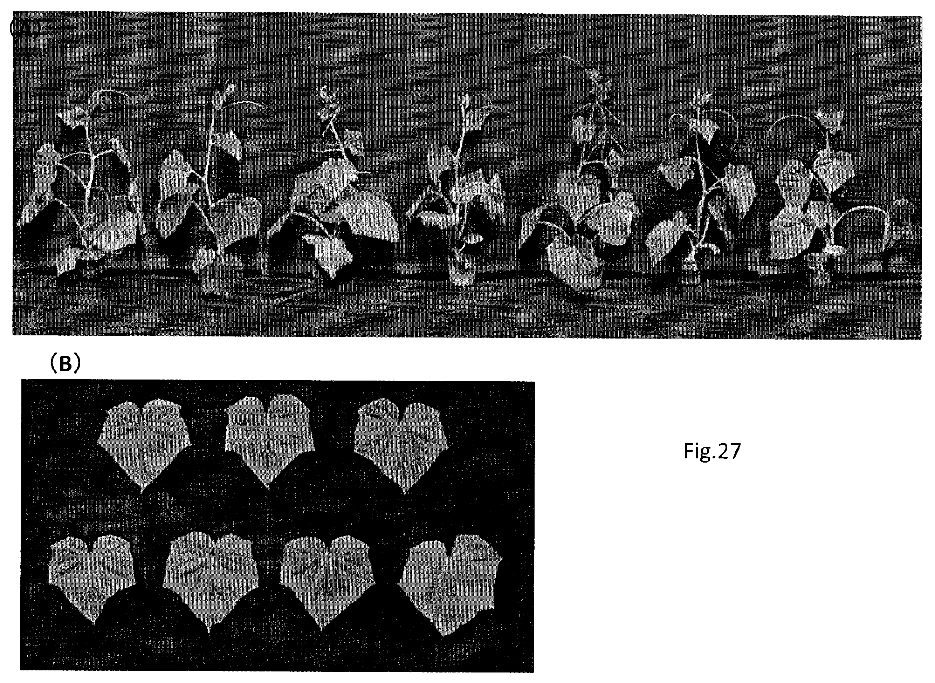

Chlorotic spots were observed in the second true leaves of all individuals after about 3 days from the secondary inoculation, and in the subsequently developed upper leaves in the test group that had the secondary inoculation of only CMV-42CM (CMV-42CM only group), and in the test group that had the secondary inoculation of CMV-42CM after the primary inoculation of wtALSV (wtALSV+CMV-42CM group). Specifically, mosaic symptoms were observed in the sixth to eighth true leaves. In contrast, no disease symptoms were observed in any of the individuals in the test group that had the secondary inoculation of CMV-42CM after the primary inoculation of ALSV-C:2a (ALSV-C:2a+CMV-42CM group). Two of the six individuals showed chlorotic spots in the inoculated leaves, and in the second to fourth true leaves in the test group that had the secondary inoculation of CMV-42CM after the primary inoculation of ALSV-C:1a (ALSV-C:2a+CMV-42CM group). No disease symptoms were observed in the fifth and subsequent true leaves. The remaining four individuals did not show disease symptoms. The same disease symptoms observed in the CMV-42CM only group and the wtALSV+CMV-42CM group were observed in all of the six individuals of the test group that had the secondary inoculation of CMV-42CM after the primary inoculation of ALSV-C:CP (ALSV-C:CP+CMV-42CM group) (FIG. 27).

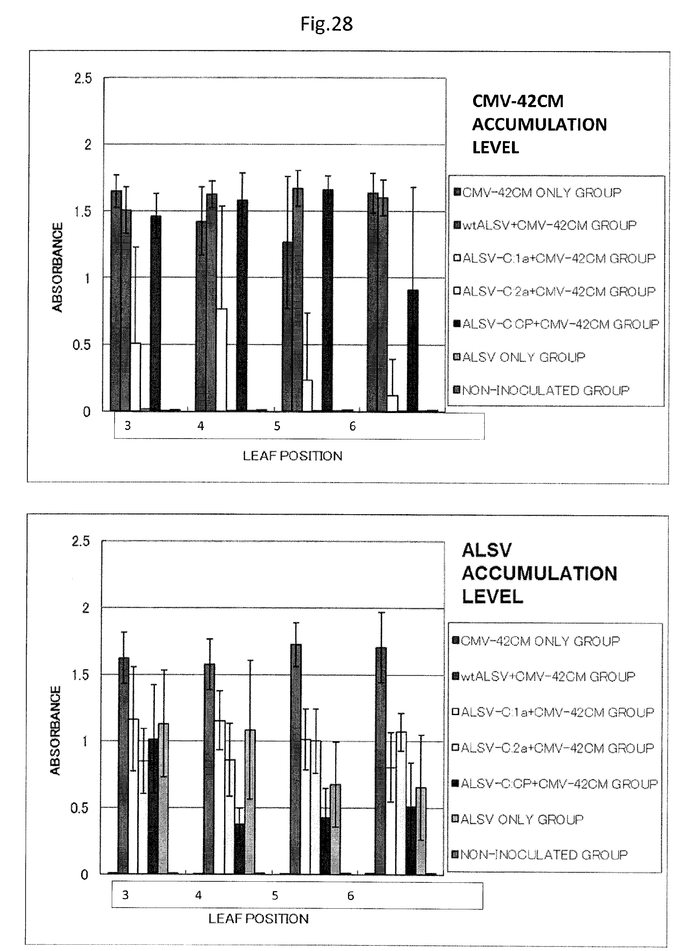

A CMV-42CM assay confirmed no CMV-42CM accumulation in the ALSV-C:2a+CMV-42CM group. In contrast, the ALSV-C:CP+CMV-42CM group had the same levels of CMV-42CM accumulation observed in the CMV-42CM only group and the wtALSV+CMV-42CM group, and ZYMV accumulated in about half the amount in the ALSV-C:1a+CMV-42CM group (FIG. 28). The ALSV-C:1a+CMV-42CM group and the ALSV-C:2a+CMV-42CM group had the same levels of ALSV accumulation observed in the ALSV only group, and the wtALSV+CMV-42CM group had increased levels of accumulation compared to the ALSV only group. On the other hand, the accumulation in the ALSV-C:CP+CMV-42CM group was less than that of the ALSV only group (FIG. 28). The ALSVs (ALSV-C:1a, ALSV-C:2a, ALSV-C:CP) containing the introduced CMV-Y gene fragments used for the primary inoculation were checked for insertion. The inserts were retained in the all ALSVs.

### 3: Discussion

The interference effect against CMV-Y and CMV-42CM were induced, and disease symptoms and virus accumulation were not confirmed in the test groups in which the ALSV-C:2a of the tobacco and cucumber plants were inoculated. On the other hand, mild disease symptoms were observed, and CMV-Y and CMV-42CM accumulations were confirmed in the test groups that had the ALSV-C:1a and ALSV-C:CP inoculation. The difference in the interference effect is believed to be due to the different gene fragments introduced to the ALSV vectors.

The absence of the CMV-Y and CMV-42CM disease symptoms and virus accumulation in the ALSV-C:2a containing the 2a gene fragment suggests that the interference effect by the ALSV vector is also inducible in tobacco and cucumber, and the ALSV vector has potential use as an interference effect inducible vector in ALSV vector host plants.

By comparing the homology of the nucleotide sequences in the CMV-Y gene fragment regions introduced to ALSV and the corresponding CMV-42CM gene regions, it was found that the 1 a gene, the 2a gene, and the CP gene were 90.3%, 98.1%, and 97.9% homologous, respectively.

In Example 2, the interference effect against two CMVs (CMV-Y and CMV-42CM) was induced by the 2a gene fragment-introduced ALSV constructed from the known CMV-Y gene. This result suggests that interference effects against different strains or isolates could be induced by inoculating a host with an ALSV vector containing a fragment of a gene region having a conserved homologous nucleotide sequence.

Taken together with the result of Example 1, the successful induction of interference effect with the ALSV vector in the viruses of different genera suggests that protective agents (plant vaccines) that induce interference effect against different viruses can be created in short time periods by changing the gene fragment introduced to the ALSV vector.

### Example 3

### Interference Effect of ALSV Vector with Introduced SMV Gene Fragment

An ALSV (SMVCP-ALSV) was created by inserting a part of the genome sequence (201 nt) coding for the SMV coat protein (CP), and tested for interference effect against SMV. The SMVCP-ALSV was inoculated to the cotyledons of soybean varieties (Jack, and Tanbaguro) in the primary inoculation, and SMV was inoculated to the developed primary leaf or first true leaf by using the silicon carbide method (secondary inoculation, or challenge inoculation).

In contrast to the SMV sole inoculation group that showed mosaic and wrinkling symptoms in the upper leaves, no disease symptoms were observed in the group that had the primary inoculation of SMVCP-ALSV. The SMV proliferation was considerably weaker than in the SMV sole inoculation group, confirming the interference effect by the SMVCP-ALSV. A tissue blot analysis of the SMV distribution in upper leaves revealed that SMV was distributed in mosaic patterns over the whole leaf surface in individuals that had inoculation of only SMV, and that the viruses were localized in the veins and the surrounding areas in individuals that had primary inoculation of SMVCP-ALSV.

### Example 4

### Interference Effect of ALSV Vector with Introduced ZYMV and CMV Gene Fragments

An ALSV (ZC-ALSV) was created by inserting a fused gene fragment of the ZYMV gene fragment (150 nt) used in Example 1 and the CMV gene fragment (150 nt) of Example 2, and tested for interference effect against ZYMV and CMV. The ZC-ALSV was inoculated to the cotyledons of a cucumber as primary inoculation. After 5 to 6 days, ZYMV and CMV were inoculated to the first true leaf either individually or as a mixture (secondary inoculation, or challenge inoculation).

Severe symptoms were observed in the control groups (the ZYMV secondary inoculation group had severe ZYMV mosaic involving deformation in the upper leaves; the CMV secondary inoculation group had severe mosaic; the mixed secondary inoculation group had severe mosaic and dwarfing symptoms). In contrast, the ZC-ALSV primary inoculation group showed interference effect in all of the secondary inoculation groups inoculated with ZYMV alone and CMV alone and together. Further, no clear disease symptoms were confirmed, and virus accumulation was suppressed.

These results demonstrate that it is possible to create an ALSV combination vaccine strain that simultaneously suppresses two types of viruses. **Sequence Listing Free Text**

## Claims

1. A recombinant apple latent spherical virus (ALSV) comprising a partial fragment of a genomic RNA or cDNA from one or more plant pathogenic virus.

2. The recombinant ALSV of claim 1, wherein the plant pathogenic virus is a zucchini yellow mosaic virus (ZYMV).

3. The recombinant ALSV of claim 1, wherein the plant pathogenic virus is a cucumber mosaic virus (CMV).

4. The recombinant ALSV of claim 1, wherein the plant pathogenic virus is asoybean mosaic virus (SMV).

5. A method for defending against infection of a plant pathogenic virus, which comprises inoculating the recombinant ALSV of claim 1 into a seedling of a plant.

6. A pathogenic virus-resistant plant, which is inoculated with the recombinant ALSV in a seedling stage.


SEQUENCE LISTING

<110> Iwate University

<120> A recombinant ALSV preventing plant viral infection

<130> 11F016PCT

<140> PCT/JP2011/061099
<141> 2011-05-13

<150> JP 2011-066704
<151> 2011-03-24

<160> 21

<210> 1
<211> 32
<212> DNA
<213> Artificial

<220>
<223> Description of artificial sequence: Synthetic oligonucleotide

<400> 1
tacatctcga ggcaagtagt cgggttggta ta 32

<210> 2
<211> 33
<212> DNA
<213> Artificial

<220>
<223> Description of artificial sequence: Synthetic oligonucleotide

<400> 2
tacatggatc ctccattcgg accctttttc acc 33

<210> 3
<211> 32
<212> DNA
<213> Artificial

<220>
<223> Description of artificial sequence: Synthetic oligonucleotide

<400> 3
tacatctcga ggcggcgaca agggaagaaa aa 32

<210> 4
<211> 30
<212> DNA
<213> Artificial

<220>
<223> Description of artificial sequence: Synthetic oligonucleotide

<400> 4
tacatggatc ctgcgccagc catgtacaag 30

<210> 5
<211> 33
<212> DNA
<213> Artificial

<220>
<223> Description of artificial sequence: Synthetic oligonucleotide

<400> 5
tacatctcga gggctccggc tcaggtgaga aaa 33

<210> 6
<211> 31
<212> DNA
<213> Artificial

<220>
<223> Description of artificial sequence: Synthetic oligonucleotide

<400> 6
tacatggatc cagaggcgaa ttgctgatga g 31

<210> 7
<211> 31
<212> DNA
<213> Artificial

<220>
<223> Description of artificial sequence: Synthetic oligonucleotide

<400> 7
tacatggatc ccaatgacat tttctttgtg a 31

<210> 8
<211> 15
<212> DNA
<213> Artificial

<220>
<223> Description of artificial sequence: Synthetic oligonucleotide

<400> 8
gcgaggcact cctta 15

<210> 9
<211> 15
<212> DNA

<213>  Artificial

<220>
<223>  Description of artificial sequence: Synthetic oligonucleotide

<400>  9
gcaaggtggt cgtga                                        15

<210>  10
<211>  20
<212>  DNA
<213>  Artificial

<220>
<223>  Description of artificial sequence: Synthetic oligonucleotide

<400>  10
atggcgacgt cctcgttcaa                                   20

<210>  11
<211>  20
<212>  DNA
<213>  Artificial

<220>
<223>  Description of artificial sequence: Synthetic oligonucleotide

<400>  11
accaaccagt ggtgtgacgt                                   20

<210>  12
<211>  20
<212>  DNA
<213>  Artificial

<220>
<223>  Description of artificial sequence: Synthetic oligonucleotide

<400>  12
gcattctcac tagccaatct                                   20

<210>  13
<211>  20
<212>  DNA
<213>  Artificial

<220>
<223>  Description of artificial sequence: Synthetic oligonucleotide

<400>  13
atcatcagcg aaagtcctct                                   20

<210>  14
<211>  20

<212> DNA
<213> Artificial

<220>
<223> Description of artificial sequence: Synthetic oligonucleotide

<400> 14
tctgaatcaa ccagtgctgg 20

<210> 15
<211> 20
<212> DNA
<213> Artificial

<220>
<223> Description of artificial sequence: Synthetic oligonucleotide

<400> 15
tgaatacacg aggacggcgt 20

<210> 16
<211> 30
<212> DNA
<213> Artificial

<220>
<223> Description of artificial sequence: Synthetic oligonucleotide

<400> 16
aagacaactc atgagcaact cgaggagcaa 30

<210> 17
<211> 30
<212> DNA
<213> Artificial

<220>
<223> Description of artificial sequence: Synthetic oligonucleotide

<400> 17
cgcggatcct agacaatcga gagttccaca 30

<210> 18
<211> 30
<212> DNA
<213> Artificial

<220>
<223> Description of artificial sequence: Synthetic oligonucleotide

<400> 18
ccgctcgagg atgagtttgt aacttatggt 30

<210> 19

<211>  30
<212>  DNA
<213>  Artificial

<220>
<223>  Description of artificial sequence: Synthetic oligonucleotide

<400>  19
cgcggatcct tcaaaacact tcatggttcg                                30

<210>  20
<211>  30
<212>  DNA
<213>  Artificial14:53 2013/09/19

<220>
<223>  Description of artificial sequence: Synthetic oligonucleotide

<400>  20
ccgctcgaga aaatagaccg tgggtcttat                                30

<210>  21
<211>  30
<212>  DNA
<213>  Artificial

<220>
<223>  Description of artificial sequence: Synthetic oligonucleotide

<400>  21
cgcggatcca gatgcagcat actgataaac                                30